# EUROPEAN PATENT SPECIFICATION

(11) **EP 1 370 568 B1**
(45) Date of publication and mention of the grant of the patent: **12.10.2005**
(21) Application number: 02714332.0
(22) Date of filing: 19.03.2002
(51) Int. Cl.: C07H 19/16, C07D 473/18, C07D 473/34, A61K 31/70, A61P 9/10, A61P 25/00

(54) **CHEMICAL COMPOUNDS**
CHEMISCHE VERBINDUNGEN
COMPOSES CHIMIQUES

(30) Priority: 20.03.2001 GB 0106877; 20.03.2001 GB 0106875; 20.03.2001 GB 0106871
(43) Date of publication of application: 17.12.2003
(73) Proprietor: GLAXO GROUP LIMITED, Greenford, Middlesex UB6 ONN (GB)
(72) Inventor: HALL, Adrian, GlaxoSmithKline, Welwyn, Hertfordshire AL6 9AR (GB); JANDU, Karamjit Singh, GlaxoSmithKline, Welwyn, Hertfordshire AL6 9AR (GB); LUNNIS, Christopher James, GlaxoSmithKline, Stevenage, Hertfordshire SG1 2NY (GB); VINADER, Maria Victoria, GlaxoSmithKline, Stevenage, Hertfordshire SG1 2NY (GB); WEST, Robert Ian, GlaxoSmithKline, Stevenage, Hertfordshire SG1 2NY (GB)
(74) Representative: Giddings, Peter John
(86) International application number: PCT/GB2002/001317
(87) International publication number: WO 2002/074780

(56) References cited:
- EP-A- 0 334 361
- WO-A-99/24449
- WO-A-99/38877
- WO-A-99/67262

## Description

### Field of the Invention

The present invention is concerned with pharmaceutical compositions containing certain adenosine derivatives having an acetylene group in the 4' position, which are adenosine A1 agonists, and to their use in therapy. In particular, it is concerned with the use of these adenosine derivatives in treating conditions where there is an advantage in decreasing plasma free fatty acid concentration, or reducing heart rate or which subject is suffering from or susceptible to ischaemic heart disease, peripheral vascular disease or stroke or which subject is suffering pain, a CNS disorder, sleep apnoea or emesis.

### Background of the Invention

A variety of compounds which are agonists at the adenosine A1 receptor have been described in the art. These include compounds described in published patent applications WO99/24449, WO99/24450, WO99/24451, WO97/43300, WO98/16539, WO98/04126, WO98/01459, EP0322242, GB2226027, EP222330, WO98/08855, WO94/0707, WO99/67262 and WO00/23447.

For example, WO 99/67262 (Glaxo Group Limited) discloses compounds of formula (I) which are agonists at the adenosine A1 receptor and their use in treating a patient suffering from a condition where there is an advantage in decreasing plasma free fatty acid concentration, or reducing heart rate or which subject is suffering from or susceptible to ischaemic heart disease, peripheral vascular disease or stroke or which subject is suffering pain, a CNS disorder or sleep apnoea: 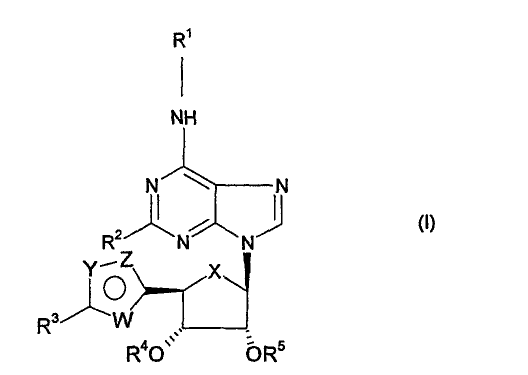 wherein Y and Z represent O, N, CH, N(C₁₋₆ alkyl);
W represents CH, O, N, S, N(C₁₋₆ alkyl);
and wherein at least one of W and Z represents a heteroatom (and when Y, Z and/or W is N, the presence or absence of an additional H would be apparent to a person skilled in the art);
with the proviso that when W represents CH, Z represents N and Y represents O, R³ cannot be H.

The compounds disclosed in WO99/67262 (Glaxo Group Limited) are made, for example, via intermediates which have an acetylene group in the 4' position.

The present inventors have surprisingly found that adenosine derivatives with an acetylene group in the 4' position exhibit Adenosine A1 agonist activity.

### Summary of the Invention

The present invention provides compounds of formula (Ia): 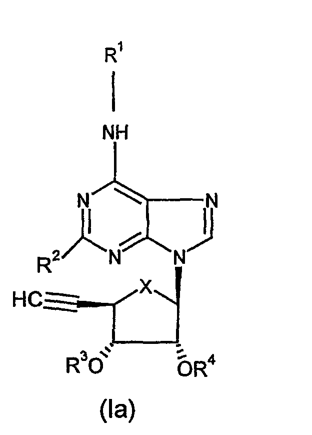 wherein X represents O or CH₂;
R¹ represents:
(i) -(alk)ₙ-(C₃₋₉)cycloalkyl or -(alk)ₙ-(C₃₋₉)cycloalkenyl, said cycloalkyl or cycloalkenyl group being optionally substituted by one or more substituents selected from OH, halogen, C₁₋₆alkyl, -C₁₋₆alkoxy, C₂₋₆alkenyloxy-, C₂₋₆alkynyloxy- and phenyl, wherein (alk) represents C₁₋₃alkyl and n represents 0 or 1, and said (alk) group may be optionally substituted by a C₃₋₆cycloalkyl group;
(ii) a phenyl group optionally substituted by one or more substituents selected from: halogen, CF₃, cyano, -C₁₋₆alkyl, -C₂₋₆alkenyl, -C₂₋₆alkynyl, C₁₋₆alkoxy-, -C₁₋₆alkylOH, -CO₂H and -CO₂C₁₋₆ alkyl;
(iii) a C₄₋₇heterocyclic group containing at least one heteroatom selected from O, N or S, and optionally substituted by one or more substituents selected from: OH, -C₁₋₆alkyl, -C₁₋₆alkoxy, -CO₂C₁₋₄alkyl, -COC₁₋₄alkyl, -CO₂aryl or - CO₂(alk)ₙ(C₃₋₆)cycloalkyl, wherein (alk) represents C₁₋₃alkyl and n represents 0 or 1;
(iv) a straight or branched C₁₋₁₂alkyl group optionally substituted by one or more groups selected from phenyl, halogen, hydroxy, and C₃₋₇ cycloalkyl, wherein one or more carbon atoms of the C₁₋₁₂alkyl group may be optionally replaced by a group independently selected from S(=O)ₙ (where n is 0, 1 or 2) and N;
(v) a fused bicyclic ring 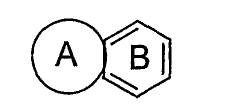
wherein A represents C₄₋₆cycloalkyl or phenyl and B represents phenyl optionally substituted by C₁₋₃alkyl, and the bicyclic ring is attached to the purine-6-amino moiety via a ring atom of ring A;
R² represents -C₁₋₃alkyl, halogen, hydrogen or C₁₋₃alkoxy group;
R³ and R⁴ independently represent H or a -C₁₋₆alkyl group;
or pharmaceutically acceptable derivatives thereof.

Further aspects of the invention are:
- A pharmaceutical composition comprising a compound of formula (Ia) or a pharmaceutically acceptable derivative thereof together with a pharmaceutical carrier and/or excipient.
- Use of a compound of formula (Ia) or a pharmaceutically acceptable derivative thereof for the manufacture of a medicament for the treatment of a patient suffering from a condition where there is an advantage in decreasing plasma free fatty acid concentration, or reducing heart.
- Use of a compound of formula (Ia) or a pharmaceutically acceptable derivative thereof for the manufacture of a medicament for the treatment of a patient suffering from or susceptible to ischaemic heart disease, peripheral vascular disease or stroke or which subject is suffering pain, a CNS disorder, sleep apnoea or emesis.
- A method of treating a patient suffering from a condition where there is an advantage in decreasing plasma free fatty acid concentration, or reducing heart rate comprising administering a therapeutically effective amount of a compound of formula (Ia) or a pharmaceutically acceptable derivative thereof.
- A method of treating a patient suffering or susceptible to ischaemic heart disease, peripheral vascular disease or stroke or which subject is suffering pain, a CNS disorder, sleep apnoea, or emesis comprising administering a therapeutically effective amount of a compound of formula (Ia) or a pharmaceutically acceptable derivative thereof.

### Detailed Description of the Invention

The compounds useful in the invention are agonists at the adenosine A1 receptor. Preferably they are selective agonists at the adenosine A1 receptor. By selective is meant that the affinity for the A1 receptor is at least 2 times, preferably 5 times and more preferably 10 times greater than the other adenosine receptor subtypes, particularly A3. Agonist selectivity of compounds against other human adenosine receptors can be determined using Chinese hamster ovary (CHO) cells transfected with the gene for the relevant human adenosine receptor following a method based on that of Castanon, KV. and Spevak, W. (1994) *Biochem. Biophys. Res. Commun.* 198, 626-631. The CHO cells are also transfected with cyclic AMP response elements promoting the gene for secreted placental alkaline phosphatase (SPAP) (Wood, KV. (1995) *Curr. Opinion. Biotechnology,* 6, 50-58). The effect of test compounds is determined by their effects on basal levels of cAMP (A2a) or on forskolin-enhanced cAMP (A1 and A3) as reflected by changes in levels of SPAP. EC₅₀ values for compounds are determined as a ratio to that of the non-selective agonist N-ethyl carboxamidoadenosine (NECA). "Adenosine receptors: New opportunities for future drugs" (S.A. Poulsen *et al*, *Bioorg. Med. Chem*,. 1998, 6, 619-641) summarises disease conditions that may be treated with adenosine A1 agonists.

The compounds of formula (Ia) contain chiral (asymmetric) centres. The individual stereoisomers (enantiomers and diastereoisomers) and mixtures of these are within the scope of the present invention.

As used herein, the terms "alkyl" and "alkoxy" mean both straight and branched chain saturated hydrocarbon groups. Examples of alkyl groups include methyl, ethyl, propyl and butyl groups. Examples of alkoxy groups include methoxy and ethoxy groups. Other examples include propoxy and butoxy. Alkyl groups may be unsubstituted, or substituted with one to four substituents, preferably one to three substituents as defined hereinabove.

One to three, preferably one or two, carbon atoms of an alkyl chain may be replaced by a group independently selected from S(=O)ₙ (where n is 0, 1 or 2) and N. When the heteroatom N replaces a carbon atom in a C₁₋₁₂alkyl group the N atom will, where appropriate be substituted by one or two substituents selected from hydrogen and C₁₋₆alkyl.

As used herein, the terms "alkenyl", "alkenyloxy", "alkynyl" and "alkynyloxy" mean both straight and branched chain unsaturated hydrocarbon groups. Examples of alkenyl groups include ethylene and propylene. Examples of alkynyl groups include ethynyl and propynyl. Examples of alkenyloxy groups include propenyloxy and ethenyloxy. Examples of alkynyloxy groups include propynyloxy and ethylyloxy.

As used herein, the term "halogen" means fluorine, chlorine, bromine or iodine.

As used herein, the term "aryl" means monocyclic or bicyclic aromatic carbocyclic groups such as phenyl and naphthyl, especially phenyl.

As used herein, the term "cycloalkyl" means an aliphatic group having 3 to 9 carbon atoms in the ring system unless otherwise defined. The cycloalkyl group can be monocyclic or bicyclic. A bicyclic group may be fused or bridged. Examples of monocyclic cycloalkyl groups include cyclopropyl, cyclobutyl and cyclopentyl. Other examples of monocyclic cycloalkyl groups are cyclohexyl, cycloheptyl and cyclooctyl. Examples of bicyclic cycloalkyl groups include bicyclo[2.2.1]hept-2-yl. Preferably, the cycloalkyl group is monocyclic. Cycloalkyl groups may be unsubstituted, or substituted with one to four substituents, preferably one or two substituents as defined hereinabove.

As used herein, the term "cycloalkenyl" means a partially unsaturated aliphatic group having 3 to 9 carbon atoms in the ring system. The cycloalkenyl group can be monocyclic or bicyclic. Preferably, the cycloalkyl group is monocyclic. Examples of monocyclic cycloalkenyl groups include cyclopentenyl and cyclohexenyl. Cycloalkenyl groups may be unsubstituted, or substituted with one to four substituents, preferably one or two substituents.

As used herein, the term "heterocyclic" means a cyclic group of 4 to 7 carbon atoms wherein one or more of the carbon atoms is/are replaced by heteroatoms independently selected from nitrogen, oxygen or sulfur. The heterocycle may be aromatic or non-aromatic, i.e., may be saturated (i.e. aliphatic), partially or fully unsaturated. This group may optionally be substituted as defined hereinabove. The heteroatom is preferably O or N. The heterocycle is preferably non-aromatic. Examples of heterocyclyl groups include piperidinyl, tetrahydrofuranyl and tetrahydropyranyl. Cycloalkyl groups may be unsubstituted, or substituted with one to four substituents, preferably one or two substituents. Heterocyclyl groups may be unsubstituted, or substituted with one to four substituents, preferably one or two substituents.

As used herein, the term "pharmaceutically acceptable derivative", means any pharmaceutically acceptable salt, solvate, ester or amide, or salt or solvate of such ester or amide, of the adenosine A1 agonist, or any other compound which upon administration to the recipient is capable of providing (directly or indirectly) the adenosine A1 agonist or an active metabolite or residue thereof, e.g. a prodrug. Pharmaceutically acceptable derivatives according to the invention are pharmaceutically acceptable salts and solvates.

As used herein, the term "pharmaceutically acceptable" means a compound which is suitable for pharmaceutical use.

Pharmaceutically acceptable salts of the compounds of formula (Ia) include those derived from pharmaceutically acceptable inorganic and organic acids. Examples of suitable acids include hydrochloric, hydrobromic, sulphuric, nitric, perchloric, fumaric, maleic, phosphoric, glycollic, lactic, salicylic, succinic, toluene-p-sulphonic, tartaric, acetic, citric, methanesulfonic, formic, benzoic, malonic, naphthalene-2-sulfonic and benzenesulfonic acids. Other acids such as oxalic, while not in themselves pharmaceutically acceptable, may be useful as intermediates in obtaining the compounds of the invention and their pharmaceutically acceptable acid addition salts.

Those skilled in the art of organic chemistry will appreciate that many organic compounds can form complexes with solvents in which they are reacted or from which they are precipitated or crystallized. These complexes are known as "solvates". For example, a complex with water is known as a "hydrate". Solvates of the compound of formula (Ia) are within the scope of the invention.

As used herein, the term "prodrug" means a compound which is converted within the body, e.g. by hydrolysis in the blood, into its active form that has medical effects. Pharmaceutically acceptable prodrugs are described in T. Higuchi and V. Stella, *Prodrugs* as *Novel Delivery Systems*, Vol. 14 of the A.C.S. Symposium Series; and in Edward B. Roche, ed., *Bioreversible Carriers in Drug Design,* American Pharmaceutical Association and Pergamon Press, 1987.

In an alternative aspect R¹ represents:
(i) -(alk)ₙ-(C₃₋₉)cycloalkyl, including bridged cycloalkyl, optionally substituted by one or more substituents selected from OH, halogen, C₁₋₃alkoxy- and phenyl, wherein (alk) represents C₁₋₆alkyl and n represents 0 or 1;
(ii) a phenyl group optionally substituted by one or more substituents selected from: halogen, CF₃, cyano, -C₁₋₆alkyl, -C₂₋₆alkenyl, -C₂₋₆alkynyl, C₁₋₆ alkoxy-, -C₁₋₆alkylOH, -CO₂H and -CO₂C₁₋₆ alkyl;
(iii) a C₄₋₇heterocyclic group containing at least one heteroatom selected from O, N or S, and optionally substituted by one or more substituents selected from: OH, -C₁₋₆alkyl, -C₁₋₆alkoxy, -CO₂(C₁₋₄)alkyl, -CO(C₁₋₄)alkyl or -CO₂aryl;
(iv) a straight or branched C₁₋₁₂alkyl substituted by one or more groups selected from: S(=O)ₙ (where n is 0, 1 or 2) substituted within the alkyl chain, N substituted within the alkyl chain, phenyl, halogen, hydroxy or C₃₋₇cycloalkyl;
(v) a fused bicyclic ring 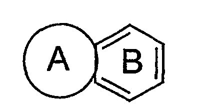
wherein A represents C₄₋₆cycloalkyl or phenyl and B represents phenyl optionally substituted by C₁₋₃alkyl, and the bicyclic ring is attached to the nitrogen atom of formula (I) via a ring atom of ring A.

In a further aspect R¹ represents:
(i) -(alk)ₙ-(C₃₋₉)cycloalkyl, said cycloalkyl group being optionally substituted by one or more substituents selected from: OH, halogen, -C₁₋₆ alkyl, C₁₋₆ alkoxy-, and phenyl, wherein (alk) represents C₁₋₃alkyl and n represents 0 or 1;
(ii) a C₄₋₇aliphatic heterocyclic group containing at least one heteroatom selected from O, N or S, optionally substituted by one or more substituents selected from: -C₁₋₆alkyl, -CO(C₁₋₄)alkyl, -CO₂(C₁₋₄)alkyl, and -CO₂phenyl;
(iii) a straight or branched C₁₋₁₂alkyl group optionally substituted by one or more groups selected from: phenyl, S(=O)ₙ (where n is 0, 1 or 2) and N wherein each S(=O)ₙ or N replaces a carbon of the alkyl group.

In a further aspect R¹ represents a phenyl group optionally substituted by one or more substituents selected from: halogen, CF₃, cyano, -C₁₋₆alkyl, -C₂₋₆alkenyl, - (C₂₋₆)alkynyl, (C₁₋₆)alkoxy-, -(C₁₋₆)alky/OH, -CO₂H and -CO₂(C₁₋₆)alkyl.

In a yet further aspect R¹ represents phenyl optionally substituted by one or more substituents selected from: halogen and -C₁₋₆alkyl; R² represents hydrogen or halogen; and R³ and R⁴ independently represent H or -C₁₋₆alkyl.

Conveniently, R¹ may represent (alk)ₙ-(C₃₋₉)cycloalkyl wherein n is 0 or 1 and the said cycloalkyl is either unsubstituted or substituted by at least one substituent selected from -C₁₋₆alkyl, C₁₋₆alkoxy-, phenyl and OH. Alternative substituents include at least one substituent selected from halogen, and C₂₋₆ alkenyloxy-. Preferably the cycloalkyl group is unsubstituted or monosubstituted by C₁₋₃alkyloxy-, C₂₋₃alkenyloxy-, -C₃₋₆cycloalkyl, C₁₋₃ alkyl, phenyl or OH, or is mono- or disubstituted by halogen, for example fluorine. Alternatively the cycloalkyl group is unsubstituted or substituted with OH or C₁₋₃ alkyl. Preferably n is zero. Preferably the cycloalkyl ring has 3 to 8 carbon atoms, more preferably 5 or 6 carbon atoms. Cycloalkyl groups include hydroxycyclopentyl or methoxycyclohexyl. Other cycloalkyl groups include propenyloxycyclohexyl, difluorocyclohexyl, ethoxycyclohexyl, dicyclopropylmethyl, cyclooctyl and cycloheptyl. When n is 1, the (alk) group may be substituted, for example by cyclopropyl.

R¹ may represent (alk)ₙ-(C₃₋₉)cycloalkenyl wherein n is 0 or 1 and the said cycloalkenyl is either substituted by at least one substituent selected from -C_{1- 6}alkyl, C₁₋₆alkoxy-, phenyl and OH or is unsubstituted. Alternative substituents include at least one substituent selected from halogen, -C₂₋₆ alkenyloxy, and -C₃₋₆cycloalkyl. Preferably n is zero. More preferably, the cycloalkenyl group is unsubstituted. Preferably the cycloalkenyl ring has 5 or 6 carbon atoms, more preferably the ring is cyclohexenyl.

Alternatively, R¹ may represent a substituted or unsubstituted aliphatic heterocyclic group, the substitutent being selected from C₁₋₆alkyl, -CO₂(C₁₋₄)alkyl or -CO₂phenyl. The substituent may also be -CO₂(alk)ₙ(C₃₋₆)cycloalkyl. Preferably the heterocyclic ring is 6 membered and more preferably contains only one O or N heteroatom. Conveniently, the aliphatic heterocyclic group is unsubstituted or, when substituted, the substituent is -CO₂(C₁₋₄)alkyl or - CO₂(alk)ₙ(C₃₋₆)cycloalkyl, the heteroatom is N and the substituent is directly attached to said ring nitrogen atom. Preferably when the heterocycle is substituted the substituent is -CO₂(C₁₋₄)alkyl, the heteroatom is N and the substituent is directly attached to said ring nitrogen atom. Most preferably when the heterocyclic ring is unsubstituted the heteroatom is O. Most preferably when the heterocyclic ring is substituted the heteroatom is N.

Alternatively, R¹ may represent a straight or branched alkyl of 1-6 carbon atoms including one or more S(=O)ₙ groups (where n is 0, 1 or 2) each replacing a carbon atom of the alkyl group, optionally substituted with N replacing a carbon atom at a position adjacent to the S(=O)ₙ group. Preferably n is 1 or 2, more preferably n is 2.

Alternatively, R¹ may represent phenyl optionally substituted one or two substitutents selected from halogen or C₁₋₆alkyl. More preferably, R¹ preferably represents phenyl optionally substituted one or two substitutents selected from halogen or methyl. Preferably the halogen is fluorine, chlorine or bromine, more preferably fluorine or chlorine. Preferably the phenyl is disubstituted. Preferably the phenyl is disubstituted in the 2,3 or 2,4 or 2,5 positions. In an alternative aspect the phenyl is monosubstituted by C₁₋₆ alkyl, for example methyl.

In a preferred aspect of the invention, R¹ represents:
(i) -(alk)ₙ(C₃₋₉) cycloalkyl, said cycloalkyl group being optionally substituted by one or more substituents selected from OH, halogen, C₁₋₆alkyl, -C_{1- 6}alkoxy, C₂₋₆alkenyloxy- and phenyl, wherein (alk) represents C₁₋₃alkyl and n represents 0 or 1, and said (alk) group may be optionally substituted by a C₃₋₆cycloalkyl group; or R¹ represents (alk)ₙ-(C_{3- 9})cycloalkenyl;
(ii) a C₄₋₇heterocyclic group containing at least one heteroatom selected from O or N, optionally substituted by -C₁₋₆alkyl, -CO₂C₁₋₄alkyl, or - CO₂(alk)ₙ(C₃₋₆)cycloalkyl; or
(iii) phenyl optionally substituted by halogen or C₁₋₆alkyl.

In an alternative aspect of the invention, R¹ represents:
(i) -(alk)ₙ(C₃₋₉) cycloalkyl, including bridged cycloalkyl, said cycloalkyl group being optionally substituted by one or more substituents selected from OH, C₁₋₆ alkyl, C₁₋₆ alkoxy, and phenyl, wherein (alk) represents C₁₋₃alkyl and n represents 0 or 1;
(ii) a C₄₋₇ heterocyclic group containing at least one heteroatom selected from O or N, optionally substituted by -C₁₋₆alkyl, or -CO₂C₁₋₄alkyl; or
(iii) phenyl optionally substituted by halogen or C₁₋₆alkyl.

In another aspect of the invention, R¹ represents:
(i) -(alk)ₙ-(C₃₋₉)cycloalkyl, including bridged cycloalkyl, said cycloalkyl group optionally substituted by one or more substituents selected from OH, -C_{1- 6}alkyl, -C₁₋₆alkoxy, and phenyl; wherein (alk) represents C₁₋₃alkyl and n represents 0 or 1; or
(ii) a C₄₋₇ aliphatic heterocyclic group containing at least one heteroatom selected from O or N, optionally substituted by -C₁₋₆alkyl or -CO₂C₁₋₄alkyl.

R² preferably represents hydrogen or halogen. More preferably, R² represents hydrogen or chlorine. Most preferably, R² represents hydrogen.

R³ and R⁴ preferably both represent hydrogen.

X preferably represents O.

When R¹ is C₁₋₁₂alkyl, the group is preferably substituted.

It is to be understood that the present invention covers all combinations of particular and preferred groups mentioned above.

Preferred compounds of the invention include:
(2R,3R,4S,5R)-2-{6-[(4-chloro-2-fluorophenyl)amino]-9H-purin-9-yl}-5-ethynyltetrahydrofuran-3,4-diol;
(2R,3R,4S,5R)-2-{2-chloro-6-[(4-chloro-2-fluorophenyl)amino]-9H-purin-9-yl}-5-ethynyltetrahydrofuran-3,4-diol;
(2R,3R,4S,5R)-2-{6-[(2-chloro-4-fluorophenyl)amino]-9H-purin-9-yl}-5-ethynyltetrahydrofuran-3,4-diol;
(2R,3R,4S,5R)-5-ethynyl-2-{6-[(4-fluoro-2-methylphenyl)amino]-9H-purin-9-yl}tetrahydrofuran-3,4-diol;
(2R,3R,4S,5R)-2-{6-[(2,4-difluorophenyl)amino]-9H-purin-9-yl}-5-ethynyltetrahydrofuran-3,4-diol;
(2R,3R,4S,5R)-5-ethynyl-2-{6-[(3-fluoro-2-methylphenyl)amino]-9H-purin-9-yl}tetrahydrofuran-3,4-diol;
(2R,3R,4S,5R)-2-{6-[(3-chloro-2-methylphenyl)amino]-9H-purin-9-yl}-5-ethynyltetrahydrofuran-3,4-diol;
(2R,3R,4S,5R)-2-{6-[(4-chloro-2-methylphenyl)amino]-9H-purin-9-yl}-5-ethynyltetrahydrofuran-3,4-diol;
(2R,3R,4S,5R)-2-{6-[(5-chloro-2-methylphenyl)amino]-9H-purin-9-yl}-5-ethynyltetrahydrofuran-3,4-diol;
(2R,3R,4S,5R)-2-{6-[(2-bromo-4-fluorophenyl)amino]-9H-purin-9-yl}-5-ethynyltetrahydrofuran-3,4-diol;
(2R,3R,4S,5R)-2-{2-chloro-6-[(3-fluoro-2-methylphenyl)amino]-9H-purin-9-yl}-5-ethynyltetrahydrofuran-3,4-diol;
(2R,3R,4S,5R)-2-{6-[(3,4-difluorophenyl)amino]-9H-purin-9-yl}-5-ethynyltetrahydrofuran-3,4-diol;
N-ethyl-2-({9-[(2R,3R,4S,5R)-5-ethynyl-3,4-dihydroxytetrahydrofuran-2-yl]-9H-purin-6-yl}amino)ethanesulfonamide;
ethyl 4-({9-[(2R,3R,4S,5R)-5-ethynyl-3,4-dihydroxytetrahydrofuran-2-yl]-9H-purin-6-yl}amino)piperidine-1-carboxylate;
(2R,3R,4S,5R)-5-ethynyl-2-(6-{[(1S,2S)-2-hydroxycyclopentyl]amino}-9H-purin-9-yl)tetrahydrofuran-3,4-diol;
(2R,3R,4S,5R)-2-[6-indan-2-ylamino)-9H-purin-9-yl]-5-ethynyltetrahydrofuran-3,4-diol;
(2 R,3R,4S,5R)-2-[6-(cyclobutylamino)-9H-purin-9-yl]-5-ethynyltetrahydrofu ran-3,4-diol;
(2R,3R,4S,5R)-5-ethynyl-2-{6-[(2-phenylethyl)amino]-9H-purin-9-yl}tetrahydrofuran-3,4-diol;
(2R,3R,4S,5R)-2-[6-(cyclohexylamino)-9H-purin-9-yl]-5-ethynyltetrahydrofuran-3,4-diol;
(2R,3R,4S,5R)-5-ethynyl-2-(6-{[(1S*,2S*,3S*,4R*)-3-methylbicyclo[2.2.1]hept-2-yl]amino}-9H-purin-9-yl)tetrahydrofuran-3,4-diol;
(2R,3R,4S,5R)-5-ethynyl-2-(6-{[(1R*,*2S)-2-methoxy-2-phenylcyclopentyl]amino}-9H-purin-9-yl)tetrahydrofuran-3,4-diol;
(2R, 3R,4S,5R)-2-[6-(cyclopropylamino)-9H-purin-9-yl]-5-ethynyltetrahydrofuran-3,4-diol;
(2R,3R,4S,5R)-2-{6-[(cyclopropylmethyl)amino]-9H-purin-9-yl}-5-ethynyltetrahydrofuran-3,4-diol;
(2R,3R,4S,5R)-2-[6-(cyclopentylamino)-9H-purin-9-yl]-5-ethynyltetrahydrofuran-3,4-diol;
(2R,3R,4S,5R)-5-ethynyl-2-(6-{[(2R,3R)-2-methyltetrahydrofuran-3-yl]amino}-9H-purin-9-yl)tetrahydrofuran-3,4-diol;
(2R,3R,4S,5R)-5-ethynyl-2-(6-{[(2S,3S)-2-methyltetrahydrofuran-3-yl]amino}-9H-purin-9-yl)tetrahydrofuran-3,4-diol;
(2R,3R,4S,5R)-5-ethynyl-2-{6-[(trans-4-methoxycyclohexyl)amino]-9H-purin-9-yl}tetrahydrofuran-3,4-diol;
2R,3R,4S,5R)-5-ethynyl-2-{6-[(trans-4-ethoxycyclohexyl)amino]-9H-purin-9-yl}tetrahydrofuran-3,4-diol;
2R,3R,4S,5R)-5-ethynyl-2-{6-[(trans-4-propenyloxycyclohexyl)amino]-9H-purin-9-yl}tetrahydrofuran-3,4-diol;
(2R,3R,4S,5R)-5-ethynyl-2-[6-(tetrahydro-2H-pyran-4-ylamino)-9H-purin-9-yl]tetrahydrofuran-3,4-diol;
(2R,3R,4S,5R)-2-{6-[(1R*,5R*,6S*)-bicyclo[3.2.0]hept-6-ylamino]-9H-purin-9-yl}-5-ethynyltetrahydrofuran-3,4-diol;
(2R,3R,4S,5R)-2-{6-[(2,2-dimethylcyclopropyl)amino]-9H-purin-9-yl}-5-ethynyltetrahydrofuran-3,4-diol;
(2R,3R,4S,5R)-5-ethynyl-2-{6-[(trans-4-hydroxycyclohexyl)amino]-9H-purin-9-yl}tetrahydrofuran-3,4-diol;
(2R,3R,4S,5R)-2-{6-[(2-methylphenyl)amino]-9H-purin-9-yl}-5-ethynyltetrahydrofuran-3,4-diol;
(2R,3R,4S,5R)-2-{6-[(4-methylphenyl)amino]-9H-purin-9-yl}-5-ethynyltetrahydrofuran-3,4-diol;
(2R,3R,4S,5R)-2-{6-[(3-chloro-2-fluorophenyl)amino]-9H-purin-9-yl}-5-ethynyltetrahydrofuran-3,4-diol;
(2R,3R,4S,5R)-2-{6-[(2-fluoro-5-methylphenyl)amino]-9H-purin-9-yl}-5-ethynyltetrahydrofuran-3,4-diol;
butyl 4-({9-[(2R,3R,4S,5R)-5-ethynyl-3,4-dihydroxytetrahydrofuran-2-yl]-9H-purin-6-yl}amino)piperidine-1-carboxylate;
cyclopropylmethyl 4-({9-[(2R,3R,4S,5R)-5-ethynyl-3,4-dihydroxytetrahydrofuran-2-yl]-9H-purin-6-yl}amino)piperidine-1-carboxylate;
(2R,3R,4S,5R)-5-ethynyl-2-{(6-[(1S,2S)-2-methoxycyclopentyl]amino}-9H-purin-9-yl)tetrahydrofuran-3,4-diol;
(2R,3R,4S,5R)-5-ethynyl-2-{6-[(4,4-difluoro)cyclohexyl]amino}-9H-purin-9-yl)tetrahydrofuran-3,4-diol;
(2R,3R,4S,5R)-5-ethynyl-2-{6-[(cyclohex-3-enyl)amino]}-9H-purin-9-yl)tetrahydrofuran-3,4-diol;
(2R,3R,4S,5R)-5-ethynyl-2-[6-(dicyclopropylmethyl)amino]-9H-purin-9-yl)tetrahydrofuran-3,4-diol;
(2R,3R,4S,5R)-5-ethynyl-2-[6-(cyclooctyl)amino]-9H-purin-9-yl)tetrahydrofuran-3,4-diol;
(2R,3R,4S,5R)-5-ethynyl-2-[6-(cycloheptyl)amino]-9H-purin-9-yl)tetrahydrofuran-3,4-diol;
(2R,3R,4S,5R)-5-ethynyl-2-{6-[(1R*,4S*)-4-methoxy-cyclohept-2-enylamino)-9H-purin-9-yl]-tetrahydrofuran-3,4-diol; or
(2R,3R,4S,5R)-5-ethynyl-2-{6-[(1S*,4R*)-4-methoxy-cyclohept-2-enylamino)-9H-purin-9-yl]-tetrahydrofuran-3,4-diol.

Particularly preferred compounds of the invention include:
(2R,3R,4S,5R)-2-{6-[(2-chloro-4-fluorophenyl)amino]-9H-purin-9-yl}-5-ethynyltetrahydrofuran-3,4-diol;
(2R,3R,4S,5R)-5-ethynyl-2-{6-[(4-fluoro-2-methylphenyl)amino]-9H-purin-9-yl}tetrahydrofuran-3,4-diol;
(2R,3R,4S,5R)-5-ethynyl-2-{6-[(3-fluoro-2-methylphenyl)amino]-9H-purin-9-yl}tetrahydrofuran-3,4-diol;
(2R,3R,4S,5R)-2-{6-[(3-chloro-2-methylphenyl)amino]-9H-purin-9-yl}-5-ethynyltetrahydrofuran-3,4-diol;
(2R,3R,4S,5R)-2-{6-[(4-chloro-2-methylphenyl)amino]-9H-purin-9-yl}-5-ethynyltetrahydrofuran-3,4-diol;
ethyl 4-({9-[(2R,3R,4S,5R)-5-ethynyl-3,4-dihydroxytetrahydrofuran-2-yl]-9H-purin-6-yl}amino)piperidine-1-carboxylate;
(2R,3R,4S,5R)-5-ethynyl-2-(6-{[(1S,2S)-2-hydroxycyclopentyl]amino}-9H-purin-9-yl)tetrahydrofuran-3,4-diol;
(2R,3R,4S,5R)-5-ethynyl-2-[6-(tetrahydro-2H-pyran-4-ylamino)-9H-purin-9-yl]tetrahydrofuran-3,4-diol;
(2R,3R,4S,5R)-5-ethynyl-2-{6-[(trans-4-methoxycyclohexyl)amino]-9H-purin-9-yl}tetrahydrofuran-3,4-diol;
2R,3R,4S,5R)-5-ethynyl-2-{6-[(trans-4-ethoxycyclohexyl)amino]-9H-purin-9-yl}tetrahydrofuran-3,4-diol;
2R,3R,4S,5R)-5-ethynyl-2-{6-[(trans-4-propenyloxycyclohexyl)amino]-9H-purin-9-yl}tetrahydrofuran-3,4-diol;
(2R,3R,4S,5R)-2-{6-[(1R*,5R*,6S*)-bicyclo[3.2.0]hept-6-ylamino]-9H-purin-9-yl}-5-ethynyltetrahydrofuran-3,4-diol; or
(2R,3R,4S,5R)-2-{6-[(2,2-dimethylcyclopropyl)amino]-9H-purin-9-yl}-5-ethynyltetrahydrofuran-3,4-diol.

Compounds according to the invention have applicability as inhibitors of lipolysis i.e. they decrease plasma free fatty acid concentrations. The compounds may thus be used in the treatment of hyperlipidaemias. Furthermore, as a consequence of their anti-lipolytic activity, the compounds have the ability to lower elevated blood glucose, insulin and ketone body levels and therefore may be of value in the therapy of diabetes. Since anti-lipolytic agents have hypolipidaemic and hypofibrinogenaemic activity, the compounds may also show anti-atherosclerotic activity. The assay described by P. Strong *et al*. in *Clinical Science* (1993), 84, 663-669 may be used to determine the anti-lipolytic activity of compounds of the invention by their ability to lower the concentration of non-esterified fatty acids (NEFA) in starved rats.

In addition to their anti-lipolytic effect, the compounds of the invention may independently affect cardiac function by reducing heart rate and conduction. The compounds may thus be used in the therapy of a number of cardiovascular disorders, for example cardiac arrythmias, particularly following myocardial infarction, and angina. The compounds may also inhibit renin release and thus be of use in the therapy of hypertension and heart failure.

Furthermore, the compounds of the invention are useful as cardioprotective agents, having applicability in the treatment of ischaemic heart disease. As used herein the term "ischaemic heart disease" includes damage associated with both myocardial ischaemia and reperfusion, for example, associated with coronary artery bypass grafting (CABG), percutaneous translumenal coronary angioplasty (PTCA), cardioplegia, acute myocardial infarction, thrombolysis, stable and unstable angina and cardiac surgery including in particular cardiac transplantation. The compounds of the invention additionally are useful for treating ischaemic damage to other organs. The compounds of the invention may also be valuable in the treatment of other disorders arising as a result of widespread atheromatous disease, for example, peripheral vascular disease (PVD) and stroke.

The compounds of the invention may also be useful as CNS agents (e.g. as hypnotics, sedatives, analgestics and/or anti-convulsants particularly finding use in the treatment of epilepsy). They are therefore useful in treating or preventing pain. They may be used to improve the condition of a host, typically of a human being, suffering from pain. They may be employed to alleviate pain in a host. Thus, a compound of formula (Ia) and its pharmaceutically acceptable acid addition salts may be used as a preemptive analgesic to treat acute pain such as musculoskeletal pain, post operative pain and surgical pain, chronic pain such as chronic inflammatory pain (e.g. rheumatoid arthritis and osteoarthritis), neuropathic pain (e.g. post herpetic neuralgia, diabetic neuropathies associated with diabetes, trigeminal neuralgia, pain associated with functional bowel disorders, e.g. irritable bowel syndrome, non cardiac chest pain and sympathetically maintained pain) and pain associated with cancer and fibromyalgia. The compound of formula (Ia) may also be used in the treatment or prevention of migraine or of pain associated with migraine, tension headache and cluster headaches, pain associated with functional bowel disorders (e.g. IBS), non cardiac chest pain and non ulcer dyspepsia. The compound of formula (Ia) may also be used in the treatment of nociceptive pain (e.g. headaches, labour pain, menstrual pain and early post-operative pain).

In addition, the compounds of the invention may find use in the treatment of sleep apnoea.

In addition, the compounds of the invention may find use in the treatment of emesis. Treatment of emesis includes treatment of nausea, retching and vomiting. Emesis includes acute emesis, delayed emesis and anticipatory emesis.

Accordingly, the invention provides a compound of formula (Ia) or a pharmaceutically acceptable derivative thereof for use in therapy, and in particular in the treatment of human or animal subjects suffering from a condition in which there is an advantage in decreasing plasma free fatty acid concentration, or reducing heart rate and conduction, or whereby the therapy involves the treatment of ischaemic heart disease, peripheral vascular disease or stroke or which subject is suffering from a pain, a CNS disorder, sleep apnoea or emesis.

In another aspect, the invention provides a method of treatment of a human or animal subject suffering from a condition in which there is an advantage in decreasing plasma free fatty acid concentration, or reducing heart rate and conduction, or which subject is suffering from or susceptible to ischaemic heart disease, peripheral vascular disease or stroke, or which subject is suffering from pain, a CNS disorder, sleep apnoea or emesis, which method comprises administering to the subject an effective amount of a compound of formula (Ia) or a pharmaceutically acceptable derivative thereof.

In another aspect the invention also provides for the use of a compound of formula (Ia) or a pharmaceutically acceptable derivative thereof for the manufacture of a medicament for the treatment of human or animal subjects suffering from a condition in which there is an advantage in decreasing plasma free fatty acid concentration, or reducing heart rate and conduction, or which subject is suffering from or susceptible to ischaemic heart disease, peripheral vascular disease (PVD) or stroke, or which patient is suffering from pain, a CNS disorder, sleep apnoea or emesis.

In respect of the above mentioned ischaemic treatment, the methods of the present invention are applicable not only where ischaemia is planned or expected, for example in cardiac surgery, but also in cases of sudden or unexpected ischaemia, for example in heart attack and unstable angina.

In a preferred aspect, the invention provides a compound of formula (Ia) or a pharmaceutically acceptable derivative thereof for use in therapy, and in particular in the treatment of human or animal subjects of conditions associated with pain including acute pain, chronic pain, inflammatory pain, neuropathic pain, nociceptive pain and pain associated with migraine, tension headaches, cluster headaches and functional bowel disorder. In an alternative embodiment, the invention provides a compound of formula (Ia) or a pharmaceutically acceptable derivative thereof for use in therapy, and in particular in the treatment of human or animal subjects of conditions associated with pain including acute pain, chronic pain, inflammatory pain, neuropathic pain and pain associated with migraine, tension headaches, cluster headaches and functional bowel disorder.

In another aspect, the invention provides a method of treatment of a human or animal subject suffering from a condition associated with pain including acute pain, chronic pain, inflammatory pain, neuropathic pain, nociceptive pain and pain associated with migraine, tension headaches, cluster headaches and functional bowel disorder; alternatively acute pain, chronic pain, inflammatory pain, neuropathic pain and pain associated with migraine, tension headaches, cluster headaches and functional bowel disorder; which method comprises administering to the subject an effective amount of a compound of formula (Ia) or a pharmaceutically acceptable derivative thereof.

In another aspect the invention also provides for the use of a compound of formula (Ia) or a pharmaceutically acceptable derivative thereof for the manufacture of a medicament for the treatment of human or animal subjects suffering from a condition associated with pain including acute pain, chronic pain, inflammatory pain, neuropathic pain, nociceptive pain and pain associated with migraine, tension headaches, cluster headaches and functional bowel disorder. In an alternative embodiment the invention provides for the use of a compound of formula (Ia) or a pharmaceutically acceptable derivative thereof for the manufacture of a medicament for the treatment of human or animal subjects suffering from a condition associated with pain including acute pain, chronic pain, inflammatory pain, neuropathic pain and pain associated with migraine, tension headaches, cluster headaches and functional bowel disorder.

It will be appreciated that reference to treatment includes acute treatment or prophylaxis as well as the alleviation of established symptoms.
While it is possible that compounds of the invention may be administered as the raw material, it is preferable to present the active ingredient as a pharmaceutical formulation.

In a further aspect, the invention provides a pharmaceutical composition comprising at least one compound of formula (Ia) or a pharmaceutically acceptable derivative thereof in association with a pharmaceutically acceptable carrier and/or excipient. The carrier and/or excipient must be "acceptable" in the sense of being compatible with the other ingredients of the formulation and not deleterious to the receipient thereof.

In another aspect, the invention provides a pharmaceutical composition comprising, as active ingredient, at least one compound of formula (Ia) or a pharmaceutically acceptable derivative thereof in association with a pharmaceutically acceptable carrier and/or excipient for use in therapy, and in particular in the treatment of human or animal subjects suffering from a condition in which there is an advantage in decreasing plasma free fatty acid concentration, or reducing heart rate and conduction, or which subject is suffering from or susceptible to ischaemic heart disease, peripheral vascular disease or stroke, or which subject is suffering from a CNS disorder, sleep apnoea, pain or emesis.

There is further provided by the present invention a process of preparing a pharmaceutical composition, which process comprises mixing at least one compound of formula (Ia) or a pharmaceutically acceptable derivative thereof, together with a pharmaceutically acceptable carrier and/or excipient.

Compositions according to the invention may be formulated for topical, oral, buccal, parenteral or rectal administration or in a form suitable for administration by inhalation or insufflation. Oral administration is preferred. The compositions may be adapted for sustained release.

For topical administration, the pharmaceutical composition may be given in the form of a transdermal patch.

Tablets and capsules for oral administration may contain conventional excipients such as binding agents, for example mucilage of starch or polyvinylpyrrolidone; fillers, for example, lactose, microcrystalline cellulose or maize-starch; lubricants, for example, magnesium stearate or stearic acid; disintegrants, for example, potato starch, croscarmellose sodium or sodium starch glycollate; or wetting agents such as sodium lauryl sulphate. The tablets may be coated according to methods well known in the art. Oral liquid preparations may be in the form of, for example, aqueous or oily suspensions, solutions, emulsions, syrups or elixirs, or may be presented as a dry product for constitution with water or other suitable vehicle before use. Such liquid preparations may contain conventional additives such as suspending agents, for example, sorbitol syrup, methyl cellulose, or carboxymethyl cellulose; emulsifying agents, for example, sorbitan mono-oleate; non-aqueous vehicles (which may include edible oils), for example, propylene glycol or ethyl alcohol; and preservatives, for example, methyl or propyl p-hydroxybenzoates or sorbic acid. The preparations may also contain buffer salts, flavouring, colouring and sweetening agents (e.g. mannitol) as appropriate.

For buccal administration the compositions may take the form of tablets or lozenges formulated in conventional manner.

The compounds of formula (Ia) or pharmaceutically acceptable derivatives thereof may be formulated for parenteral administration by bolus injection or continuous infusion and may be presented in unit dose form in ampoules, or in multi-dose containers with an added preservative. The compositions may take such forms as suspensions, solutions, or emulsions in oily or aqueous vehicles, and may contain formulatory agents such as suspending, stabilising and/or dispersing agents. Alternatively, the active ingredient may be in powder form for constitution with a suitable vehicle, e.g. sterile pyrogen-free water, before use.

The compounds of formula (Ia) or pharmaceutically acceptable derivatives thereof may also be formulated as suppositories, e.g. containing conventional suppository bases such as cocoa butter or other glycerides.
A proposed dose of the compounds of the invention for administration to man (of approximately 70kg body weight) is 0.1mg to 2g, preferably 1mg to 2g, more preferably 1 mg to 100mg, of the active ingredient per unit dose which could be administered, for example, 1 to 4 times per day. It will be appreciated that it may be necessary to make routine variations to the dosage, depending on the age and condition of the patient. The dosage will also depend on the route of administration.

The compounds of formula (Ia) may also be used in combination with other therapeutic agents. The invention thus provides, in a further aspect, a combination comprising a compound of formula (Ia) or a pharmaceutically acceptable derivative thereof together with a further therapeutic agent.

When a compound of formula (Ia) or a pharmaceutically acceptable derivative thereof is used in combination with a second therapeutic agent active against the same disease state the dose of each compound may differ from that when the compound is used alone. Suitable second therapeutic agents for the treatment of pain include, for example, COX-2 inhibitor e.g. celecoxib, rofecoxib, vaidecoxib, parecoxib, 4-(4-cyclohexyl-2-methyl-5-oxazolyl)-2-fluorobenzenesulfonamide (JTE-522), MK663, nimesulide, DFP, 2-(4-ethoxyphenyl)-3-(4-methanesulfonyl-phenyl)-pyrazolo[1,5-b]pyridazine; 5HT1 agonists e.g. sumatriptan, naratriptan, zolmitriptan, eletriptan, rizatriptan, frovatriptan, almotriptan, alniditan, dihydroergotamine, donitriptan, PNU-142633, ALX-0646, LY334370, U1092291, IS159, PNY142633; sodium channel blockers e.g. lamotrigine, R(-)-2,4-diamino-5-(2,3-dichlorophenyl)-6-fluoromethyl pyrimidine, 2,6-diamino-3-(2,3,5-trichlorophenyl)pyrazine, 5-amino-6-[2,3,5-trichlorophenyl]-1,2,4-triazine; 5HT3 antagonists e.g. alosetron; gabapentin; pregabalin; EP1 antagonists e.g. ZD6416, ZD6804; and opioids e.g. alfentanil, buprenorphine, codeine, dextropropoxyphene, diamorphine, dihydrocodeine, fentanyl, methadone, morphine, oxycodone, levorphanol, pentazocine, pethidine.
The combinations referred to above may conveniently be presented for use in the form of a pharmaceutical formulation and thus pharmaceutical formulations comprising a combination as defined above together with a pharmaceutically acceptable carrier or excipient comprise a further aspect of the invention. The individual components of such combinations may be administered either sequentially or simultaneously in separate or combined pharmaceutical formulations by any convenient route.

When administration is sequential, either the adenosine A1 agonist or the second therapeutic agent may be administered first. When administration is simultaneous, the combination may be administered either in the same or different pharmaceutical composition.

When combined in the same formulation it will be appreciated that the two compounds must be stable and compatible with each other and the other components of the formulation. When formulated separately they may be provided in any convenient formulation, conveniently in such manner as are known for such compounds in the art.

When a compound of formula (Ia) or a pharmaceutically acceptable derivative thereof is used in combination with a second therapeutic agent active against the same disease state the dose of each compound may differ from that when the compound is used alone. Appropriate doses will be readily appreciated by those skilled in the art. It will be appreciated that the amount of a compound of the invention required for use in treatment will vary with the nature of the condition being treated and the age and the condition of the patient and will be ultimately at the discretion of the attendant physician or veterinarian.

The compounds of formula (Ia) and pharmaceutlically acceptable derivatives thereof may be prepared by the processes described hereinafter, said processes constituting a further aspect of the invention. In the following description, the groups X, R¹, R² and R³ are as defined for compounds of formula (Ia) unless otherwise stated.

According to a first general process (A), a compound of formula (Ia) may be prepared by reacting a compound of formula (II): 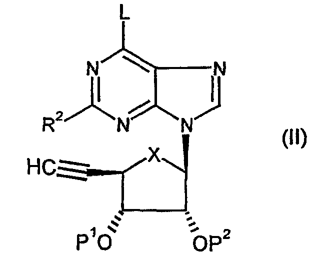 wherein L represents a leaving group such as a halogen atom (e.g. chlorine), HOBT or a linker group capable of binding to a solid phase polymeric support (e.g. a polystyrene resin) and for example may be -SO₂C₁₋₄alkylene and P¹ and P² represent hydrogen, C₁₋₆ straight chain or branched alkyl or a suitable protecting group (e.g. acetyl or a protecting group wherein P¹ and P² together form an alkylidene group) with a compound of formula R¹NH₂ or a salt thereof under basic or buffered conditions, where R¹, R² and X are as defined for compounds of formula (Ia).

Compounds of formula (II) may be used to produce compounds of formula (Ia) directly by reaction with the group R¹NH₂, where R¹ is as defined for compounds of formula (Ia), either in the absence or presence of a solvent such as an alcohol (e.g. a lower alkanol such as isopropanol, t-butanol or 3-pentanol), an ether (e.g. tetrahydrofuran or dioxan), a substituted amide (e.g. dimethylformamide), a halogenated hydrocarbon (e.g. chloroform), an aromatic hydrocarbon (e.g. toluene), dimethyl sulfoxide (DMSO) or acetonitrile, preferably at an elevated temperature (e.g. up to the reflux temperature of the solvent), in the presence of a suitable acid scavanger, for example, inorganic bases such as sodium, cesium or potassium carbonate, or organic bases such as triethylamine, diisopropylethylamine or pyridine, optionally in the presence of a palladium catalyst (e.g. palladium acetate) and phosphine ligand (e.g. R-(+/-)-2,2'-bis(diphenylphosphino)-1-1' binaphthyl).

Alternatively, compounds of formula (II) may be used to produce compounds of formula (Ia) by reaction with the group R¹NH₂, where R¹ is as defined for compounds of formula (Ia), in the presence of CaCO₃ and an appropriate solvent, e.g. ethanol or acetic acid. Preferably when the solvent is ethanol the reaction is heated, for example at reflux. In an alternative aspect the reaction may be carried out in acetic acid in the absence of CaCO₃, preferably the reaction is heated.

The above reactions may be preceded or followed where appropriate by in situ removal of the P¹ and P² protecting groups. For example when P¹ and P² represent acetyl, this may be effected with an amine such as ammonia or tert-butylamine or an alkoxide such as sodium methoxide in a solvent such as methanol or when P¹ and P² represent an alkylidene by acid hydrolysis, e.g. with trifluoroacetic acid (TFA). Interconversion of P¹ and P² protecting groups may occur at any stage in the preparation of the compounds of formula (II), for example when P¹ and P² represent acetyl, compounds of formula (II) may be prepared from compounds wherein P¹ and P² together represent an alkylidene protecting group by acid catalysed removal of the alkylidene protecting group, e.g. with hydrogen chloride in methanol followed by *in situ* acylation, for example with acetic anhydride in the presence of a base such as pyridine, in a solvent such as dichloromethane.

Otherwise, interconversion of P¹ and P² protecting groups may occur at any stage during the preparation of compounds of formula (II).

Compounds of formula (II) may be prepared by methods described in WO99/67262. For example, compounds of formula (II) where X = O may be prepared by reacting compounds of formula (III): 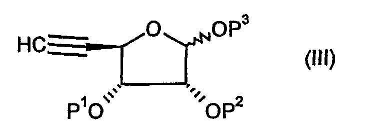 wherein P³ represents a suitable protecting group, for example acetyl, or a substituent such as C₁₋₃ alkyl, and P¹ and P² are as defined above, with compounds of formula (IV): 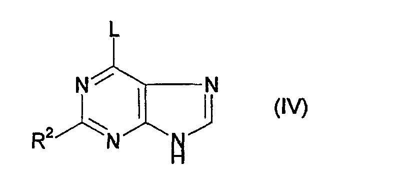 wherein L and R² are as defined above.

The reaction is conveniently carried out in a suitable solvent, such as acetonitrile in the presence of a silylating agent such as trimethylsilyl trifluoromethane sulfonate and a base such as diazabicyclo[5.4.0]undec-7-ene (DBU). Alternatively the compound of formula (IV) may first be silylated with a suitable silylating agent e.g. hexamethyldisilazane followed by reaction of the silylated intermediate with a compound of formula (III) and a suitable Lewis acid, e.g. trimethylsilyl trifluoromethanesulfonate in a suitable solvent such as acetonitrile.

Compounds of formula (IV) are either known in the art or may be prepared from known compounds using methods analogous to those used to prepare the known compounds of formula (IV).

As described above, the compounds of formula (III) may be prepared from alternative protected compounds by replacement of the alternate P¹ and P² protecting groups with other P¹ and P² groups. These represent an exchanging of one protecting group for another and will be apparent to those skilled in the art.

Compounds of formula (III) may be prepared by methods known in the art.

Specific optical isomers of a compound of formula (Ia) may be obtained by conventional methods for example, by synthesis from an appropriate asymmetric starting material using any of the processes described herein, or where appropriate by separation of a mixture of isomers of a compound of formula (Ia) by conventional means e.g. by fractional distillation, fractional crystallisation or chromatography.

In the general processes described above, the compound of formula (Ia) obtained may be in the form of a salt, conveniently in the form of a pharmaceutically acceptable salt. Where desired, such salts may be converted into the corresponding free bases using conventional methods.

Pharmaceutically acceptable acid addition salts of the compounds of formula (Ia) may be prepared by reacting a compound of formula (Ia) with an appropriate acid in the presence of a suitable solvent such as acetonitrile, acetone, chloroform, ethyl acetate or an alcohol (e.g. methanol, ethanol or isopropanol). Pharmaceutically acceptable base addition salts may be obtained in an analogous manner by treating a solution of a compound of formula (Ia) with a suitable base. Pharmaceutically acceptable salts may also be prepared from other salts, including other pharmaceutically acceptable salts of the compounds of formula (Ia), using conventional methods.

The present invention will now be further illustrated by the accompanying examples which should not be construed as limiting the scope of the invention in any way.

### Examples

Definitions of abbreviations used herein: N,N-dimethylformamide (DMF), dimethyl sulfoxide (DMSO), dichloromethane (DCM), tetrahydrofuran (THF), trifluoroacetic acid (TFA) and diazabicyclo[5.4.0]undec-7-ene (DBU).

On occasions (denoted by #) during the reaction some deprotection of the acetate intermediates may occur to give the mono and di-deprotected compounds.

### Example 1: N-Ethyl-2-({9-[(2R,3R,4S,5R)-5-ethynyl-3,4-dihydroxytetrahydrofuran-2-yl]-9H-purin-6-yl}amino)ethanesulfonamide

### Intermediate 1: (2R,3R,4R,5R)-4-(Acetyloxy)-2-[6-({2-[(ethylamino)sulfonyl]ethyl}amino)-9H-purin-9-yl]-5-ethynyltetrahydrofuran-3-yl acetate

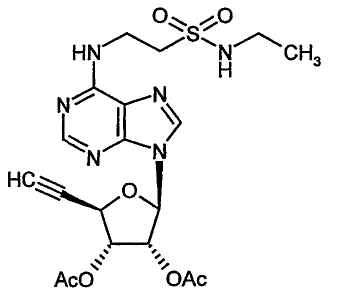

A solution containing (2R,3R,4R,5R)-4-(acetyloxy)-2-(6-chloro-9H-purin-9-yl)-5-ethynyltetrahydrofuran-3-yl acetate (0.050g), propan-2-ol (1-5ml), diisopropyethylamine(0.095ml) and 2-amino-N-ethylethanesulfonamide (0.041 g) was heated to 80°C for 3 to 18 hours. The solvent was removed *in vacuo* to give a gum. (Optional purification) Purification by automated preparative HPLC to give the title compound (0.023g) as a gum. Note on occasions (denoted by #) during the reaction some deprotection of the acetates does occur to give the mono and di-deprotected compounds.
LC/MS Rt 2.76 min.
Mass spectrum *m*/*z* 481 [MH⁺].

### Example 1: N-Ethyl-2-({9-[(2R,3R,4S,5R)-5-ethynyl-3,4-dihydroxytetrahydrofuran-2-yl]-9H-purin-6-yl}amino)ethanesulfonamide

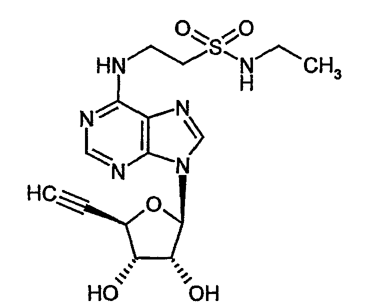

To a solution of (2R,3R,4R,5R)-4-(acetyloxy)-2-[6-({2-[(ethylamino)sulfonyl]ethyl}amino)-9H-purin-9-yl]-5-ethynyltetrahydrofuran-3-yl acetate (0.02g) in methanol (2ml) stirred at 5°C(ice bath) was added t-butylamine (0.1ml) and the mixture was stirred for 1 hour at 5°C. The solvent was removed *in vacuo.* Purification by automated preparative HPLC to give the title compound (0.012g) as a white solid
LC/MS Rt 2.29 min.
Mass spectrum *m*/*z* 397 [MH⁺].

The following compounds were made using an analogous route to the above procedure using the appropriate starting materials:

### Example 2: Ethyl 4-({9-[(2R,3R,4S,5R)-5-ethynyl-3,4-dihydroxytetrahydrofuran-2-yl]-9H-purin-6-yl}amino)piperidine-1-carboxylate

### Intermediate 2: Ethyl 4-({9-[(2R,3R,4R,5R)-3,4-bis(acetyloxy)-5-ethynyltetrahydrofuran-2-yl]-9H-purin-6-yl}amino)piperidine-1-carboxylate

Starting materials: (2R,3R,4R,5R)-4-(acetyloxy)-2-(6-chloro-9H-purin-9-yl)-5-ethynyltetrahydrofuran-3-yl acetate and ethyl-4-amino-1-piperidine carboxylate.

### Example 2: (from Intermediate 2) Ethyl 4-({9-[(2R,3R,4S,5R)-5-ethynyl-3,4-dihydroxytetrahydrofuran-2-yl]-9H-purin-6-yl}amino)piperidine-1-carboxylate

¹H NMR(MeOD) δ 8.15,8.14 (2H,br.s+s,2xCH), 6.0 (1H,d,CH),4.70(1H,t,CH) 4.60(1 H,dd,CH), 4.31 (1H,dd,CH), 4.23(1 H,vbr.m,CH), 4.07-3.98(4H,q+br.m,CH2 + 2xCH eq), 3.16(1H,d,CH), 2.98(2H,br.t, 2xCH ax), 1.96(2H,br.dd, 2xCH eq), 1.45(2H,br.dq, 2xCH ax), 1.17(3H,t,CH₃).
LC/MS(Red) Rt = 2.44min.
Mass Spectrum m/z 417 [MH+].

### Example 3: (2R,3R,S,5R)-5-Ethynyl-2-(6-{[(1S,2S)-2-hydroxycyclopentyl]amino}-9H-purin-9-yl)tetrahydrofuran-3,4-diol

### Intermediate 3: (2R,3R,4R,5R)-4-(Acetyloxy)-5-ethynyl-2-(6-{[(1S,2S)-2-hydroxycyclopentyl]amino}-9H-purin-9-yl)tetrahydrofuran-3-yl acetate

Starting materials: (2R,3R,4R,5R)-4-(acetyloxy)-2-(6-chloro-9H-purin-9-yl)-5-ethynyltetrahydrofuran-3-yl acetate and (1S, 2S)-2-aminocyclopentanol.

### Example 3: (from Intermediate 3) (2R,3R,4S,5R)-5-Ethynyl-2-(6-{[(1S,2S)-2-hydroxycyclopentyl]amino}-9H-purin-9-yl)tetrahydrofuran-3,4-diol

¹H NMR (MeOD) δ 8.29(1H,s,CH), 8.22(1H,brs,CH), 7.72(1H,br.d,NH), 5.94(1 H,d,CH), 5.78 (1Hv.br.s -OH),4.78(1H,br.m,CH) ,4.55(1H,dd,CH), 4.38(1H,t,CH), 4.25(1H,br.m, CH), 4.05(1 H,br.m, CH) ,3.75(1H,d,CH) ,3.35(2H, [H₂O],vr.br.s, 2xOH), 2.12-1.43(6H,4xm,3xCH₂).
LC/MS(Blue) Rt = 2.30min.
Mass Spectrum m/z 346 [MH+].

### Example 4: (2R,3R,4S,5R)-2-[6-(2,3-Dihydro-1H-inden-2-ylamino)-9H-purin-9-yl]-5-ethynyltetrahydrofuran-3,4-diol

### Intermediate 4: (2R,3R,4R,5R)-4-(Acetyloxy)-2-[6-(2,3-dihydro-1H-inden-2-ylamino)-9H-purin-9-yl]-5-ethynyltetrahydrofuran-3-yl acetate

Starting materials: (2R,3R,4R,5R)-4-(acetyloxy)-2-(6-chloro-9H-purin-9-yl)-5-ethynyltetrahydrofuran-3-yl acetate and 2-aminoindan.

### Example 4: (from Intermediate 4) (2R,3R,4S,5R)-2-[6-(2,3-Dihydro-1H-inden-2-ylamino)-9H-purin-9-yl]-5-ethynyltetrahydrofuran-3,4-diol

LC/MS Rt=2.65min.
*m*/*z* 378 [MH+], 376 [MH-].

### Example 5: (2R,3R,4S,5R)-2-[6-(Cyclobutylamino)-9H-purin-9-yl]-5-ethynyltetrahydrofuran-3,4-diol

### Intermediate 5: (2R,3R,4R,5R)-4-(Acetyloxy)-2-[6-(cyclobutylamino)-9H-purin-9-yl]-5-ethynyltetrahydrofuran-3-yl acetate

Starting materials: (2R,3R,4R,5R)-4-(Acetyloxy)-2-(6-chloro-9H-purin-9-yl)-5-ethynyltetrahydrofuran-3-yl acetate and cyclobutylamine.

### Example 5: (from Intermediate 5) (2R,3R,4S,5R)-2-[6-(cyclobutylamino)-9H-purin-9-yl]-5-ethynyltetrahydrofuran-3,4-diol

LC/MS Rt=2.24min.
*m*/*z* 318 [MH+].

### Example 6: (2R,3R,4S,5R)-5-Ethynyl-2-{6-[(2-phenylethyl)amino]-9H-purin-9-yl}tetrahydrofuran-3,4-diol

### Intermediate 6: (2R,3R,4R,5R)-4-(acetyloxy)-5-ethynyl-2-{6-[(2-phenylethyl)amino]-9H-purin-9-yl}tetrahydrofuran-3-yl acetate

Starting materials: (2R,3R,4R,5R)-4-(acetyloxy)-2-(6-chloro-9H-purin-9-yl)-5-ethynyltetrahydrofuran-3-yl acetate and 2-phenylethylamine.

### Example 6: (from Intermediate 6)

### (2R,3R,4S,5R)-5-ethynyl-2-{6-[(2-phenylethyl)amino]-9H-purin-9-yl}tetrahydrofuran-3,4-diol

LC/MS Rt=2.58min.
*m*/*z* 366 [MH+].

### Example 7: (2R,3R,4S,5R)-2-[6-(Cyclohexylamino)-9H-purin-9-yl]-5-ethynyltetrahydrofuran-3,4-diol

### Intermediate 7: (2R,3R,4R,5R)-4-(Acetyloxy)-5-ethynyl-2-[6-((cyclohexyl)amino]-9H-purin-9-yl}tetrahydrofuran-3-yl acetate

Starting materials: (2R,3R,4R,5R)-4-(acetyloxy)-2-(6-chloro-9H-purin-9-yl)-5-ethynyltetrahydrofuran-3-yl acetate and cyclohexylamine.

### Example 7: (from Intermediate 7) (2R,3R,4S,5R)-2-[6-(Cyclohexylamino)-9H-purin-9-yl]-5-ethynyltetrahydrofuran-3,4-diol

LC/MS Rt=2.73min.
*m*/*z* 344 [MH+].

### Example 8:# (2R,3R,4S,5R)-5-Ethynyl-2-(6-{[(1S,2S,3S,4R)-3-methylbicyclo[2.2.1]hept-2-yl]amino}-9H-purin-9-yl)tetrahydrofuran-3,4-diol

Starting materials: (2R,3R,4R,5R)-4-(acetyloxy)-2-(6-chloro-9H-purin-9-yl)-5-ethynyltetrahydrofuran-3-yl acetate and bicyclo [2.2.1]heptan-2-amine.
LC/MS Rt=2.76min.
*m*/*z* 370 [MH+].

### Example 9:# (2R,3R,4S,5R)-5-Ethynyl-2-(6-{[(1R*,2S*)-2-methoxy-2-phenylcyclopentyl]amino}-9H-purin-9-yl)tetrahydrofuran-3,4-diol

Starting materials: (2R,3R,4R,5R)-4-(acetyloxy)-2-(6-chloro-9H-purin-9-yl)-5-ethynyltetrahydrofuran-3-yl acetate and (1 R*,2S*)-2-methoxy-2-phenyl cyclopentylamine.

(1R*,2S*)-2-Methoxy-2-phenylcyclopentylamine was prepared using the following method: To a solution of 1-pheny-6-azabicyclo[3.1.0]hexane(1.2g) in methanol (30ml) was added conc. sulphuric acid (1ml) and the mixture stirred at 20°C for 5 hours. Mixture was diluted with1N sodium hydroxide (180ml) and extracted with dichloromethane (3 x 25ml). The organic phases were combined, and dried using sodium sulphate. The solvent was removed *in vacuo* to give a pale yellow oil (1.8g). (This was isolated as the maleate salt ). mpt. 132-134°C. LC/MS Rt=2.72min.
*m*/*z* 436 [MH+].

### Example 10:# (2R,3R,4S,5R)-2-[6-(Cyclopropylamino)-9H-purin-9-yl]-5-ethynyltetrahydrofuran-3,4-diol

Starting materials: (2R,3R,4R,5R)-4-(acetyloxy)-2-(6-chloro-9H-purin-9-yl)-5-ethynyltetrahydrofuran-3-yl acetate and cyclopropylamine.
LC/MS Rt=2.15min.
*m*/*z* 302 [MH+].

### Example 11:# (2R,3R,4S,5R)-2-{6-[(Cyclopropylmethyl)amino]-9H-purin-9-yl}-5-ethynyltetrahydrofuran-3,4-diol

Starting materials: (2R,3R,4R,5R)-4-(acetyloxy)-2-(6-chloro-9H-purin-9-yl)-5-ethynyltetrahydrofuran-3-yl acetate and (aminomethyl)cyclopropylamine.
LC/MS Rt=2.40min.
*m*/*z* 316 [MH+].

### Example 12:# (2R,3R,4S,5R)-2-[6-(Cyclopentylamino)-9H-purin-9-yl]-5-ethynyltetrahydrofuran-3,4-diol

Starting materials: (2R,3R,4R,5R)-4-(acetyloxy)-2-(6-chloro-9H-purin-9-yl)-5-ethynyltetrahydrofuran-3-yl acetate and cyclopentylamine.
LC/MS (blue) Rt=2.54min.
*m*/*z* 330 [MH+].

### Example 13:# (2R,3R,4S,5R)-5-Ethynyl-2-(6-{[(2R,3R)-2-methyltetrahydrofuran-3-yl]amino}-9H-purin-9-yl)tetrahydrofuran-3,4-diol

Starting materials: (2R,3R,4R,5R)-4-(acetyloxy)-2-(6-chloro-9H-purin-9-yl)-5-ethynyltetrahydrofuran-3-yl acetate and (2R,3R)-2-methyl-3-aminotetrahydrofuran.
LC/MS Rt=2.27min.
*m*/*z* 346 [MH+].

### Example 14:# (2R,3R,4S,5R)-5-Ethynyl-2-(6-{[(2S,3S)-2-methyltetrahydrofuran-3-yl]amino}-9H-purin-9-yl)tetrahydrofuran-3,4-diol

Starting materials: (2R,3R,4R,5R)-4-(acetyloxy)-2-(6-chloro-9H-purin-9-yl)-5-ethynyltetrahydrofuran-3-yl acetate and (2S, 3S)-2-methyl-3-amino tetrahydrofuran.
LC/MS Rt=2.27min.
*m*/*z* 346 [MH+].

### Example 15: (2R,3R,4S,5R)-5-Ethynyl-2-{6-[(trans-4-methoxycyclohexyl)amino]-9H-purin-9-yl}tetrahydrofuran-3,4-diol

### Intermediate 8: (2R,3R,4R,5R)-4-(Acetyloxy)-5-ethynyl-2-{6-[(trans-4-methoxycyclohexyl)amino]-9H-purin-9-yl}tetrahydrofuran-3-yl acetate

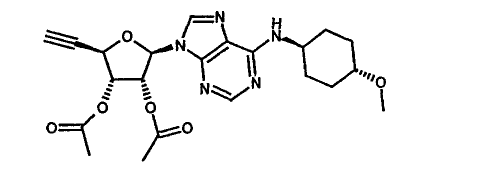

A mixture of (2R,3R,4R,5R)-4-(acetyloxy)-2-(6-chloro-9H-purin-9-yl)-5-ethynyltetrahydrofuran-3-yl acetate (6.0189g, 16.51 mmol), *N,N*-diisopropylethylamine (5ml, 28.70mmol) and trans-4-methoxy-cyclohexylamine hydrochloride (3.0011g, 18.13 mmol) was heated at reflux in iso-propanol (33ml) for 3.5hrs. The mixture was cooled to room temperature, diluted with ethyl acetate and washed with saturated ammonium chloride and saturated sodium bicarbonate then dried (sodium sulfate), filtered and concentrated. The residue was purified by chromatography using Biotage with cyclohexane containing ethyl acetate (50-100%). Evaporation gave the desired compound (4.6293g, 61 %) as a white solid.
¹H NMR (CDCl₃)δ 8.38 (s, 1 H), 8.06 (s, 1 H), 6.35(d, 1 H), 5.97(dd, 1 H) 5.68(dd, 1 H) 5.58, (brs, 1 H), 4.89(t, 1 H) 3.37 (s, 3H), 3.2 (m, 1H), 2.84(d, 1 H), 2.25-2.1 (m, 7H), 2.06 (s, 3H), 1.3-1.5 (m, 4H).

### Example 15: (2R,3R,4S,5R)-5-ethynyl-2-{6-[(trans-4-methoxycyclohexyl)amino]-9H-purin-9-yl}tetrahydrofuran-3,4-diol

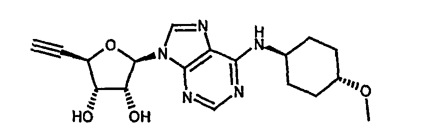

*tert*-Butylamine (5.3 ml) was added to a stirred solution of (2R,3R,4R,5R)-4-(acetyloxy)-5-ethynyl-2-{6-[(trans-4-methoxycyclohexyl)amino]-9H-purin-9-yl)tetrahydrofuran-3-yl acetate (intermediate 8) (4.6293g, 10.13 mmol) in methanol (50 ml) at room temperature. A precipitate formed. After 1 hour, the solvent was evaporated. Diethyl ether was added and decanted-off four times to leave the diol (3.5720g, 95%) as a white solid.
¹H NMR (CDCl₃) δ 8.33(s, 1 H), 8.04 (s, 1 H), 6.08 (d, 1 H), 5.97 (brs, 1 H), 5.62 (brs, 1 H), 4.82(t, 1 H), 4.71dd, 1H), 4.48(brm, 1 H), 3.37(s, 3H), 3.21(m, 1H), 2.73(d, 1 H), 2.59(m, 1 H) 2.1-2.22(m, 4H), 1.3-1.5(m, 4H).
LC/MS ([MH+] = 373, t =2.26).

### Example 16: (2R,3R,4S,5R)-5-Ethynyl-2-[6-(tetrahydro-2H-pyran-4-ylamino)-9H-purin-9-yl]tetrahydrofuran-3,4-diol

### Intermediate 9: (2R,3R,4R,5R)-4-(acetyloxy)-2-prop-1-ynyl-5-[6-(tetrahydro-2H-pyran-4-ylamino)-9H-purin-9-yl]tetrahydrofuran-3-yl acetate

Starting materials: (2R,3R,4R,5R)-4-(acetyloxy)-2-(6-chloro-9H-purin-9-yl)-5-ethynyltetrahydrofuran-3-yl acetate and 4-aminotetrahydro-2H-pyran.

### Example 16: (from Intermediate 9) (2R,3R,4S,5R)-5-ethynyl-2-[6-(tetrahydro-2H-pyran-4-ylamino)-9H-purin-9-yl]tetrahydrofuran-3,4-diol

¹H NMR (MeOD) δ 8.30d(2H,s,2xCH) 6.14d(1H,d,CH) 4.84d(1 H,t,CH) 4.75d(1 H,dd,CH) 4.40d(1 Hv.br.m,CH) 4.06d(2H,brdt,2xCHeq) 3.63d(2H,dt,2xCHax) 3.31d(1H, d,CH) 2.08d(2H,v.br, d,2xCHeq) 1.73d(2H,br.dq,2xCHax).
LC/MS Rt = 2.12min.
Mass Spectrum *m*/*z* 346 [MH+].

### Example 17:# (2R,3R,4S,5R)-2-{6-[(1R*,5R*,6S*)-Bicyclo[3.2.0]hept-6-ylamino]-9H-purin-9-yl}-5-ethynyltetrahydrofuran-3,4-diol

Starting materials: (2R,3R,4R,5R)-4-(acetyloxy)-2-(6-chloro-9H-purin-9-yl)-5-ethynyltetrahydrofuran-3-yl acetate and bicyclo[3.2.0]heptan-6-amine (Compt Rend., Ser. C, vol 263(1), 90-92 (1966)).
LC/MS Rt = 2.81 min.
Mass Spectrum *m*/*z* 356 [MH+].

### Example 18:# (2R,3R,4S,5R)-2-{6-[(2,2-Dimethylcyclopropyl)amino]-9H-purin-9-yl}-5-ethynyltetrahydrofuran-3,4-diol

Starting materials: (2R,3R,4R,5R)-4-(acetyloxy)-2-(6-chloro-9H-purin-9-yl)-5-ethynyltetrahydrofuran-3-yl acetate and 2,2-dimethoxycyclopropylamine.
LC/MS Rt = 2.5min.
Mass Spectrum *m*/*z* 330 [MH+].

### Example 19:# (2R,3R,4S,5R)-5-Ethynyl-2-{6-[(trans-4-hydroxycyclohexyl)amino]-9H-purin-9-yl}tetrahydrofuran-3,4-diol

Starting materials: (2R,3R,4R,5R)-4-(acetyloxy)-2-(6-chloro-9H-purin-9-yl)-5-ethynyltetrahydrofuran-3-yl acetate and trans-4- hydroxycyclohexylamine.
LC/MS Rt = 2.22min.
Mass Spectrum *m*/*z* 330 [MH+].

### Example 20: (2R,3R,4S,5R)-5-Ethynyl-2-{6-[(trans-4-ethoxycyclohexyl)amino]-9H-purin-9-yl}tetrahydrofuran-3,4-diol

### Intermediate 10: (2R,3R,4R,5R)-4-(acetyloxy)-5-ethynyl-2-{6-[(trans-4-ethoxycyclohexyl)amino]-9H-purin-9-yl}tetrahydrofuran-3-yl acetate

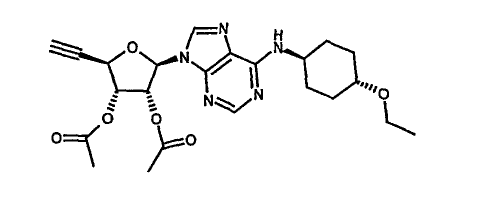

A mixture (2R,3R,4R,5R)-4-(acetyloxy)-2-(6-chloro-9H-purin-9-yl)-5-ethynyltetrahydrofuran-3-yl acetate (0.2079g 0.57 mmol), *N,N*-diisopropylethylamine (0.5ml, 2.87 mmol) and *trans*-4-ethoxycyclohexylamine hydrochloride (prepared according to JP 06329674,Takenochi *et al*), 0.1091g, 0.67 mmol) was heated in 2-propanol (1.2ml) in a reacti-vial at 90°C for 5hrs. The mixture was cooled to room temperature and evaporated. The residue was dissolved in ethyl acetate and washed with saturated ammonium chloride and saturated sodium bicarbonate then dried (sodium sulfate), filtered and concentrated. The residue was purified by chromatography using biotage with cyclohexane containing ethyl acetate (60-80%) to give the desired compound as a white solid (0.1385g, 52%).
¹H NMR (CDCl₃) δ 8.4 (brs, 1 H), 8.1 (brs, 1 H), 6.35(d, 1 H), 6.0(dd, 1 H) 5.7(dd, 1 H) 5.58, (brs, 1 H), 4. 9(t, 1H) 3.55 (q, 3H), 3.3 (m, 1H), 2.85(d, 1H), 2.25-2.1 (m, 5H ), 2.05-2.0 (m, 5H), 1.3-1.5 (t, 2H).

### Example 20: (2R,3R,4S,5R5-Ethynyl-2-{6-[(trans-4-ethoxycyclohexyl)amino]-9H-purin-9-yl}tetrahydrofuran-3,4-diol

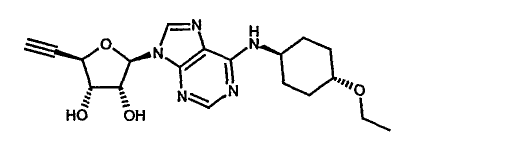

(2R,3R,4R,5R)-4-(acetyloxy)-5-ethynyl-2-{6-[(trans-4-ethoxycyclohexyl)amino]-9H-purin-9-yl}tetrahydrofuran-3-yl acetate (intermediate 22) (0.1385g, 0.29mmol) was dissolved in methanol (2.4ml) and treated with *tert*-butylamine (0.16ml) (a precipitate formed after 5 minutes). After 1 hour, the solvent was evaporated and the residue purified by Biotage with cyclohexane containing ethyl acetate (60-80%) to furnish the desired compound as a white solid ((0.1385g, 52%). ¹H NMR (CDCl₃) δ 8.33(s, 1H), 8.04 (s, 1H), 6.08 (d, 1 H), 5.97 (brs, 1 H), 5.62 (brs, 1 H), 4.82(t, 1 H), 4.71dd, 1 H), 4.48(brm, 1 H), 3.37(q, 3H), 3.21(m, 1H), 2.73(d, 1 H), 2.59(m, 1H) 2.1-2.22(m, 4H), 1.3-1.5(t, 4H).
LC-MS t=2.87

### Example 21: (2R,3R,4S,5R)-5-Ethynyl-2-{6-[(trans-4-propenyloxycyclohexyl)amino]-9H-purin-9-yl}tetrahydrofuran-3,4-diol

Starting materials: (2R,3R,4R,5R)-4-(acetyloxy)-2-(6-chloro-9H-purin-9-yl)-5-ethynyltetrahydrofuran-3-yl acetate and trans-4-propenyloxycyclohexylamine (intermediate 11)

### Intermediate 11:

(4-Hydroxy-cyclohexyl)-carbamic acid *tert*-butyl ester (1.0143g, 4.72mmol) was dissolved in THF (19 mL) and a 60% w/w suspension of sodium hydride in mineral oil (397mg, 9.94mmol) was added in portions at room temperature. Stirring was continued for 2 hours then allyl bromide (0.41 mL, 4.73mmol) was added. The mixture was stirred for a further 27 hours before quenching with 2M sodium hydroxide and extraction with ethyl acetate. The organic extracts were washed with saturated ammonium chloride then dried (Na₂SO₄), filtered and concentrated to give an oil which was purified by chromatography on Biotage with cyclohexane containing a gradient of ethyl acetate (5-15%) to yield the title compound (637mg, 53%) as a white solid.

Tlc: R_{f} 0.50 (cyclohexane-ethyl acetate 1:1)

4M Hydrochloric acid in dioxane (4mL) was added to solid (4-allyloxy-cyclohexyl)-carbamic acid *tert*-butyl ester (637mg) at room temperature and stirred for 7 hours. The solvent was evaporated to give the title compound (455mg, 95%) as a white solid .
¹H NMR (CDCl₃) δ 5.75(m, 1 H), 5.2-5.0(m, 2H), 3.9 (m, 2H), 3.2 (m, 2H), 3.0 (m, 1 H), 2.1-1.9(m, 4H), 1.4-1.1 (m, 4H).

### Example 21: (from intermediate 11) (2R,3R,4S,5R)-5-ethynyl-2-{6-[(trans-4-propenyloxycxcyclohexyl)amino]-9H-purin-9-yl}tetrahydrofuran-3,4-diol

LC/MS Rt = 2.57min.
*m*/*z* 399 [MH+].

### Example 22#: Butyl-4-({9-[(2R,3R,4S,5R)-5-ethynyl-3,4-dihydroxytetrahydrofuran-2-yl]-9H-purin-6-yl}amino)piperidine-1-carboxylate

Starting materials: (2R,3R,4R,5R)-4-(acetyloxy)-2-(6-chloro-9H-purin-9-yl)-5-ethynyltetrahydrofuran-3-yl acetate and butyl-4-amino-1-pipridine carboxylate
LC/MS Rt = 2.74min.
Mass Spectrum *m*/*z* 445 [MH+].

### Example 23#: Cyclopropylmethyl 4-({9-[(2R,3R,4S,5R)-5-ethynyl-3,4-dihydroxytetrahydrofuran-2-yl]-9H-purin-6-yl}amino)piperidine-1-carboxylate

Starting materials: (2R,3R,4R,5R)-4-(acetyloxy)-2-(6-chloro-9H-purin-9-yl)-5-ethynyltetrahydrofuran-3-yl acetate and cyclopropylmethyl-4-amino-1-piperidine carboxylate
LC/MS Rt = 2.61min.
Mass Spectrum *m*/*z* 442 [MH+].

### Example24#: (2R,3R,4S,5R)-5-Ethynyl-2-{(6-[(1S,2S)-2-methoxycyclopentyl]amino}-9H-purin-9-yl)tetrahydrofuran-3,4-diol

Starting materials: (2R,3R,4R,5R)-4-(acetyloxy)-2-(6-chloro-9H-purin-9-yl)-5-ethynyltetrahydrofuran-3-ylacetate and (1S,2S)-2-methoxycyclopentylamine
LC/MS Rt = 2.26min.
Mass Spectrum *m*/*z* 359 [MH+].

### Example25#: (2R,3R,4S,5R)-5-Ethynyl-2-{6-[(4,4-difluoro)cyclohexyl]amino}-9H-purin-9-yl)tetrahydrofuran-3,4-diol

Starting materials: (2R,3R,4R,5R)-4-(acetyloxy)-2-(6-chloro-9H-purin-9-yl)-5-ethynyltetrahydrofuran-3-ylacetate and 4,4-difluorocyclohexylamine
LC/MS Rt = 2.54min.
Mass Spectrum *m*/*z* 380[MH+].

### Example26#: (2R,3R,4S,5R)-5-Ethynyl-2-{6-[(cyclohex-3-enyl)amino]}-9H-purin-9-yl)tetrahydrofuran-3,4-diol;

Starting materials: (2R,3R,4R,5R)-4-(acetyloxy)-2-(6-chloro-9H-purin-9-yl)-5-ethynyltetrahydrofuran-3-ylacetate and cyclohex-3-enylamine
LC/MS Rt = 3.05min.
Mass Spectrum *m*/*z* 341[MH+].

### Example27#: (2R,3R,4S,5R)-5-Ethynyl-2-[6-(dicyclopropylmethyl)amino]-9H-purin-9-yl)tetrahydrofuran-3,4-diol;

Starting materials: (2R,3R,4R,5R)-4-(acetyloxy)-2-(6-chloro-9H-purin-9-yl)-5-ethynyltetrahydrofuran-3-ylacetate and dicyclopropylmethylamine
LC/MS Rt = 2.61 min.
Mass Spectrum *m*/*z* 355[MH+].

### Example28#: (2R,3R,4S,5R)-5-ethynyl-2-[6-(cyclooctyl)amino]-9H-purin-9-yl)tetrahydrofuran-3,4-diol

Starting materials: (2R,3R,4R,5R)-4-(acetyloxy)-2-(6-chloro-9H-purin-9-yl)-5-ethynyltetrahydrofuran-3-ylacetate and cyclooctylamine
LC/MS Rt = 2.80min.
Mass Spectrum *m*/*z* 371[MH+].

### Example29#: (2R,3R,4S,5R)-5-ethynyl-2-[6-(cycloheptyl)amino]-9H-purin-9-yl)tetrahydrofuran-3,4-diol

Starting materials: (2R,3R,4R,5R)-4-(acetyloxy)-2-(6-chloro-9H-purin-9-yl)-5-ethynyltetrahydrofuran-3-ylacetate and cycloheptylamine
LC/MS Rt = 2.80min.
Mass Spectrum *m*/*z* 357[MH+].

### Example 30: (2R,3R,4S,5R)-5-ethynyl-2-{6-[(1R*,4S*)-4-methoxycyclohept-2-enylamino)-9H-purin-9-yl]-tetrahydrofuran-3,4-diol

Starting materials: (2R,3R,4R,5R)-4-(acetyloxy)-2-(6-chloro-9H-purin-9-yl)-5-ethynyltetrahydrofuran-3-yl acetate and (1R*,4S*)-4-methoxy-cyclohept-2-enylamine.

Cycloheptadiene (5ml, 46mmol) was added to tetrabutylammonium periodate (20g, 46mmol) dissolved in DCM (200ml), and the mixture was cooled to 0°C. *Tert*-butyl-*N*-hydroxycarbamate (6.14g, 46mmol) was added in small portions with stirring, and the mixture was left overnight under nitrogen. The reaction mixture was washed with 10% aqueous sodium thiosulfate and saturated aqueous sodium bicarbonate. The organic layer was dried (MgSO₄) and concentrated to give a black oil. It was heated to reflux in 4% MeOH/EtOAc. On cooling tan crystals appeared which were removed by filtration. The filtrate was concentrated and purified by chromatography on silica (gradient elution EtOAc/cyclohexane, 20% to 30%). The fractions containing product were concentrated to give an oil, which was redissolved in EtOAc, washed with 10% aqueous sodium thiosulfate, dried (MgSO₄) and concentrated to give *tert*-butyl 6-oxa-7-azabicyclo[3.2.2]non-8-ene-7-carboxylate as a yellow oil (8.89g, 39.5mmol, 89%).
LC/MS Rt=2.95min.
*m*/*z* 226 [MH⁺].

*tert*-Butyl 6-oxa-7-azabicyclo[3.2.2]non-8-ene-7-carboxylate (1g, 4.44mmol) was dissolved in 10M aqueous acetic acid (15ml) and zinc dust (2.9g, 44.4mmol) was added slowly with stirring. The mixture was heated to 70°C for six hours. The cooled reaction mixture was diluted with water and ethyl acetate, filtered, and the layers separated. The aqueous layer was extracted with further ethyl acetate. The combined organic extracts were dried (NaSO₄) and concentrated to yield *cis-N*-(*tert*butyloxycarbonyl)-4-hydroxy-cyclohept-2-enylamine as a white solid (0.22g, 0.97mmol, 22%).
*m*/*z* 171 (M H⁺-*t*Bu).

Sodium hydride (66mg, 1.65mmol) was added to *cis-N-*(*tert*-butyloxycarbonyl)-4-hydroxycyclohept-2-enylamine (0.178g, 0.78mmol) dissolved in THF (3ml), and the mixture was stirred under nitrogen for one hour. Methyl iodide (48µl, 0.78mmol) was added and the mixture was stirred overnight. 2M aqueous sodium hydroxide was added to the reaction mixture it was extracted with ethyl acetate. The organic layer was washed with saturated aqueous ammonium chloride, dried (MgSO₄) and concentrated to give an orange oil. Purification by chromatography on silica (gradient elution EtOAc/cyclohexane, 10% to 50%) gave *cis*-*N*-(*tert*butyloxycarbonyl)-4-methoxy-cyclohept-2-enylamine as a white solid (79mg, 0.33mmol, 42%).
*m*/*z* 242 (MH⁺).

*cis-N-*(*tert*-Butyloxycarbonyl)-4-methoxy-cyclohept-2-enylamine (175mg, 0.73mmol) was treated with 4M hydrochloric acid in dioxane (3ml, 12mmol) and stirred at room temperature for three hours. The mixture was concentrated *in vacuo* to give a quantitative yield of the hydrochloride salt of *cis*-4-methoxycyclohept-2-enylamine as a light brown solid.
*m*/*z* 142 (MH⁺).

### Intermediate 12: (2R,3R,4R,5R)-4-(acetyloxy)-2-{6-[(1R*,4S*)-4-methoxy-cyclohept-2-enylamino)9H-purin-9-yl)-5-ethynyltetrahydrofuran-3-ylacetate

Starting materials:(2R,3R,4R,5R)-4-(acetyloxy)-2-(6-chloro-9H-purin-9-yl)-5-ethynyltetrahydrofuran-3-yl acetate and *cis*-4-methoxy-cyclophept-2-enylamine
LC/MS Rt=2.98min.
*m*/*z* 470 [MH⁺].

### Example 30: (from intermediate 12) (2R,3R,4S,5R)-5-ethynyl-2-{6-[(1R*,4S*)-4-methoxy-cyclohept-2-enylamino)-9H-purin-9-yl]-tetrahydrofuran-3,4-diol

¹H NMR (MeOD) δ 8.14(2H,s,2xCH), 5.99(1 H,d,CH), 5.76 and 5.67(2x1H, 2xbr.d, CH=CH), 4.70(1 H,t,CH), 4.60(1 H,dd,CH), 4.31(1H,t,CH), 3.93(1H,br.d, CH), 3.28(3H,s,OCH₃), 3.17(1H, d, CH), 1.93-1.33(6H,4xm,3xCH₂).
LC/MS Rt = 2.80min.
Mass Spectrum *m*/*z* 357[MH+].

### Example 31: (2R,3R,4S,5R)-5-ethynyl-2-{6-[(1S*,4R*)-4-methoxy-cyclohept-2-ylamino)-9H-purin-9-yl]-tetrahydrofuran-3,4-diol

Starting materials: (2R,3R,4R,5R)-4-(acetyloxy)-2-(6-chloro-9H-purin-9-yl)-5-ethynyltetrahydrofuran-3-ylacetate and (1 R*,4S*)-4-methoxycycloheptylamine

*tert*-Butyl 6-oxa-7-azabicyclo[3.2.2]non-8-ene-7-carboxylate (3g, 13.3mmol) was dissolved in ethanol (135ml) and added to palladium hydroxide on activated charcoal (300mg). The reaction mixture was stirred under a hydrogen atmosphere until ca. 300ml of hydrogen had been absorbed. The catalyst was removed by filtration through celite, and the organic layer was concentrated to yield *tert*-butyl 6-oxa-7-azabicyclo[3.2.2]nonane-7-carboxylate as an oil (2.98g, 13.1mmol, 98%).
*m*/*z* 228 (MH⁺).

A solution of *tert*butyl 6-oxa-7-azabicyclo[3.2.2]nonane-7-carboxylate (1.81g, 8mmol) in dry THF (25ml) under nitrogen was treated with a 0.1 M samarium diiodide in THF (250ml, 25mmol). The dark blue reaction mixture was stirred at room temperature for five hours, then diluted with DCM and quenched with 10% aqueous sodium thiosulfate (150ml). The layers were separated and the aqueous solution was extracted with DCM. The combined DCM solutions were washed with brine, and dried (NaSO₄) to give a brown solid. Purification by silica chromatography (50% EtOAc/cyclohexane) gave *cis-N*-(*tert*butyloxycarbonyl)-4-hydroxy-cycloheptylamine as a white solid (0.9g, 3.9mmol, 49%).
*m*/*z* =174 (MH⁺- *t*Bu).

Sodium hydride (332mg, 8.30mmol) was added slowly to *N*-(*tert*-butyloxycarbonyl)-4-hydroxy-cycloheptylamine (905 mg, 3.95mmol) dissolved in dry THF (16ml) under nitrogen. The mixture was stirred at room temperature for one hour. Methyl iodide (243 µl, 3.91 mmol) was added and the mixture was stirred overnight at room temperature. 2.0M aqueous sodium hydroxide was added, and the aqueous layer was extracted with ethyl acetate. The organic phase was washed with saturated aqueous ammonium chloride, dried (MgSO₄) and concentrated. The resulting oil was purified by silica chromatography (gradient elution EtOAc/cyclohexane, 15% to 50%) to yield cis-*N*-(*tert*-butyloxycarbonyl)-4-methoxy-cycloheptylamine as a white solid (290mg, 1.19mmol, 30%).

The cis-*N-*(*tert*-butyloxycarbonyl)-4-methoxy-cycloheptylamine (288mg, 1.19mmol) was dissolved in 4M hydrochloric acid in dioxane (2ml, 8mmol) and stirred at room temperature for three hours. The mixture was concentrated *in vacuo* to give a quantitative yield of the hydrochloride salt of *cis*-4-methoxyheptylamine as a yellow solid.
*m*/*z* 144 (MH⁺).

### Intermediate 13: (2R,3R,4R,5R)-4-(acetyloxy)-2-{6-[(1R*,4S*)-4-methoxy-cycloheptylamino)9H-purin-9-yl)-5-ethynyltetrahydrofuran-3-ylacetate

starting materials:(2R,3R,4R,5R)-4-(acetyloxy)-2-(6-chloro-9H-purin-9-yl)-5-ethynyltetrahydrofuran-3-yl acetate and *cis*-4-methoxy-cyclopheptylamine

### Example 31: (from intermediate 13) (2R,3R,4S,5R)-5-ethynyl-2-{6-[(1S*,4R*)-4-methoxy-cyclohept-2-ylamino)-9H-purin-9-yl]-tetrahydrofuran-3,4-diol)

¹H NMR (MeOD) δ 8.13(2H,s,2xCH), 5.99(1H,d,CH), 4.69(1H,t,CH), 4.60(1H,dd,CH), 4.30(1H,t,CH), 4.20(1H, vbr.s, NH), 3.37-3.44(1H,m, CH), 3.23(3H,s,OCH₃), 3.16(1H,d,CH), 2.05-1.30(10H,4xm,5xCH₂).
MS *m*/*z* 359[MH+]

### Example 32: (2R,3R,4S,5R)-2-{6-[(2,4-Difluorophenyl)amino]-9H-purin-9-yl}-5-ethynyltetrahydrofuran-3,4-diol

### Intermediate 14: (2R,3R,4R,5R)-4-(Acetyloxy)-2-{6-[(2,4-difluorophenyl)amino]-9H-purin-9-yl}-5-ethynyltetrahydrofuran-3-yl acetate

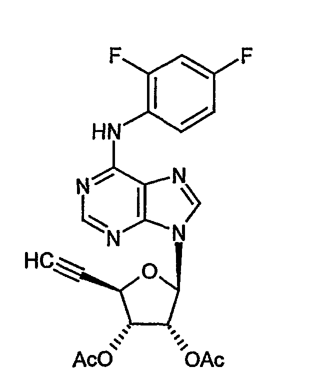

To a solution of (2R,3R,4R,5R)-4-(acetyloxy)-2-(6-chloro-9H-purin-9-yl)-5-ethynyltetrahydrofuran-3-yl acetate (0.050g) in ethanol (2ml) was added 2,4-difluroaniline (0.041ml) and calcium carbonate (0.027g). The mixture was heated to 90°C for 18 hours in a sealed vessel. The solvent was removed in vacuo. Purification by automated preparative HPLC to give the title compound (0.014 g) as a gum.

Note on some occasions deprotection of the acetates does occur to give the mono and di-deprotected compounds.
LC/MS Rt 3.25 min.
Mass spectrum *m*/*z* 457 [MH⁺].

### Example 32: (from Intermediate 14) (2R,3R,4S,5R)-2-{6-[(2,4-Difluorophenyl)amino]-9H-purin-9-yl}-5-ethynyltetrahydrofuran-3,4-diol

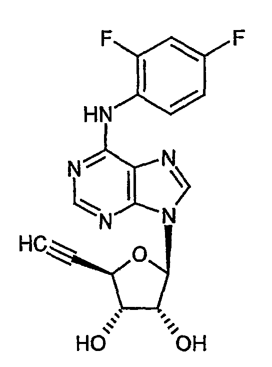

To a solution of (2R,3R,4R,5R)-4-(acetyloxy)-2-{6-[(2,4-difluorophenyl)amino]-9H-purin-9-yl}-5-ethynyltetrahydrofuran-3-yl acetate (0.012g) in methanol (2ml) stirred at 5°C (ice bath) was added t-butylamine (0.1ml) and the mixture was stirred for 1 hour at 5°C. The solvent was removed *in vacuo.* Purification by automated preparative HPLC to give the title compound (0.007g) as a gum. LC/MS Rt =2.75 min.
Mass spectrum *m*/*z* 374 [MH⁺].

The following compounds were made using an analogous route to the above procedure for Example 20 using the appropriate starting materials:

### Example 33: (2R,3R,4S,5R)-2-{6-[(4-Cloro-2-fluorophenyl)amino]-9H-purin-9-yl}-5-ethynyltetrahydrofuran-3,4-diol

### Intermediate 15: (2R,3R,4R,5R)-4-(Acetyloxy)-2-[6-(4-chloro-2-fluoroanilino)-9H-purin-9-yl]-5-ethynyltetrahydrofuran-3-yl acetate

Starting materials: (2R,3R,4R,5R)-4-(acetyloxy)-2-(6-chloro-9H-purin-9-yl)-5-ethynyltetrahydrofuran-3-yl acetate and 4-chloro-2-fluoroaniline.

### Example 33: (from Intermediate 15) (2R,3R,4S,5R)-2-{6-[(4-Chloro-2-fluorophenyl)amino]-9H-purin-9-yl}-5-ethynyltetrahydrofuran-3,4-diol

LC/MS Rt = 3.01 min.
Mass Spectrum *m*/*z* 390 [MH+].

### Example 34: (2R,3R,4S,5R)-2-{2-Chloro-6-[(4-chloro-2-fluorophenyl)amino]-9H-purin-9-yl}-5-ethynyltetrahydrofuran-3,4-diol

### Intermediate 16: (2R,3R,4R,5R)-4-(acetyloxy)-2-{2-chloro-6-[(4-chloro-2-fluorophenyl)amino]-9H-purin-9-yl}-5-ethynyltetrahydrofuran-3-yl acetate

Starting materials(2R,3R,4R,5R)-4-(acetyloxy)-2-(6-chloro-9H-purin-9-yl)-5-ethynyltetrahydrofuran-3-yl acetate and 4-chloro-2-fluoroaniline.

### Example 34: (from Intermediate 16) (2R,3R,4S,5R)-2-{2-chloro-6-[(4-chloro-2-fluorophenyl)amino]-9H-purin-9-yl}-5-ethynyltetrahydrofuran-3,4-diol

LC/MS Rt = 3.34min.
Mass Spectrum *m*/*z* 425 [MH+].

### Example 35: (2R,3R,4S,5R)-2-{6-[(2-Chloro-4-fluorophenyl)amino]-9H-purin-9-yl}-5-ethynyltetrahydrofuran-3,4-diol

### Intermediate 17: (2R,3R,4R,5R)-4-(acetyloxy)-2-{6-[(2-chloro-4-fluorophenyl)amino]-9H-purin-9-yl}-5-ethynyltetrahydrofuran-3-yl acetate

Starting materials: (2R,3R,4R,5R)-4-(acetyloxy)-2-(6-chloro-9H-purin-9-yl)-5-ethynyltetrahydrofuran-3-yl acetate and 2-chloro-4-fluoroaniline.

### Example 35: (from Intermediate 17) (2R,3R,4S,5R)-2-{6-[(2-chloro-4-fluorophenyl)amino]-9H-purin-9-yl}-5-ethynyltetrahydrofuran-3,4-diol

LC/MS Rt = 2.87min.
Mass Spectrum *m*/*z* 390 [MH+].

### Example 36: (2R,3R,4S,5R)-5-Ethynyl-2-{6-[(4-fluoro-2-methylphenyl)amino]-9H-purin-9-yl}tetrahydrofuran-3,4-diol

### Intermediate 18: (2R,3R,4R,5R)-4-(Acetyloxy)-5-ethynyl-2-{6-[(4-fluoro-2-methylphenyl)amino]-9H-purin-9-yl}tetrahydrofuran-3-yl acetate

Starting materials: (2R,3R,4R,5R)-4-(acetyloxy)-2-(6-chloro-9H-purin-9-yl)-5-ethynyltetrahydrofuran-3-yl acetate and 4-fluoro-2-methylaniline.

### Example 36: (from Intermediate 18) (2R,3S,4R,5R)-5-Ethynyl-2-{6-[(4-fluoro-2-methylphenyl)amino]-9H-purin-9-yl}tetrahydrofuran-3,4-diol

LC/MS Rt = 2.69min.
Mass Spectrum *m*/*z* 370 [MH+].

### Example 37: (2R,3R,4S,5R)-5-Ethynyl-2-{6-[(3-fluoro-2-methylphenyl)amino]-9H-purin-9-yl}tetrahydrofuran-3,4-diol

### Intermediate 19: (2R,3R,4R,5R)-4-(Acetyloxy)-5-ethynyl-2-{6-[(3-fluoro-2-methylphenyl)amino]-9H-purin-9-yl}tetrahydrofuran-3-yl acetate

Starting materials: (2R,3R,4R,5R)-4-(acetyloxy)-2-(6-chloro-9H-purin-9-yl)-5-ethynyltetrahydrofuran-3-yl acetate and 3-fluoro-2-methylaniline.

### Example 37: (from Intermediate 19) (2R,3R,4S,5R)-5-ethynyl-2-{6-[(3-fluoro-2-methylphenyl)amino]-9H-purin-9-yl}tetrahydrofuran-3,4-diol

¹H NMR (DMSO) δ 9.72 (1H,s,NH); 8.42 (1H,s,CH); 8.26 (1H,s,CH); 7.26-7.20 (2H,m,2xCH); 7.07 (1 H,m,CH); 5.99 (1 H,d,CH); 5.75 (2H,vr.s.,2xOH); 4.82 (1 H,t,CH); 4.58 (1 H,dd,CH); 4.39 (1H,t,CH); 3.77 (1H,d,CH); 2.09 (3H,d,CH₃). LC/MS Rt = 2.64min.
Mass Spectrum *m*/*z* 370 [MH+].

### Example 38: (2R,3R,4S,5R)-2-{6-[(3-Chloro-2-methylphenyl)amino]-9H-purin-9-yl}-5-ethynyltetrahydrofuran-3,4-diol

### Intermediate 20: (2R,3R,4R,5R)-4-(Acetyloxy)-2-{6-[(3-chloro-2-methylphenyl)amino]-9H-purin-9-yl}-5-ethynyltetrahydrofuran-3-yl acetate

Starting materials: (2R,3R,4R,5R)-4-(acetyloxy)-2-(6-chloro-9H-purin-9-yl)-5-ethynyltetrahydrofuran-3-yl acetate and 3-chloro-2-methylaniline.

### Example 38: (from Intermediate 20) (2R,3R,4S,5R)-2-{6-[(3-Chloro-2-methylphenyl)amino]-9H-purin-9-yl}-5-ethynyltetrahydrofuran-3,4-diol

¹H NMR (DMSO) δ 9.70 (1H,s,NH); 8.42 (1H,s,CH); 8.25 (1H,s,CH); 7.35-7.32 (2H,m,2xCH); 7.25 (1 H,t,CH); 5.99 (1 H,d,CH); 5.78 (0.5H,d,OH); 5.74 (0.5H,d,OH); 4.82 (1H,m,CH); 4.58 (1H,dd,CH); 4.38 (1H,q,CH); 3.76 (1 H,d,CH); 2.21 (3H,s,CH₃).
LC/MS Rt = 2.80min
Mass Spectrum *m*/*z* 386 [MH+]

### Example 39: (2R,3R,4S,5R)-2-{6-[(4-Chloro-2-methylphenyl)amino]-9H-purin-9-yl}-5-ethynyltetrahydrofuran-3,4-diol

### Intermediate 21: (2R,3R,4R,5R)-4-(Acetyloxy)-2-{6-[(4-chloro-2-methylphenyl)amino]-9H-purin-9-yl}-5-ethynyltetrahydrofuran-3-yl acetate

Starting materials: (2R,3R,4R,5R)-4-(acetyloxy)-2-(6-chloro-9H-purin-9-yl)-5-ethynyltetrahydrofuran-3-yl acetate and 4-chloro-2-methylaniline.

### Example 39: (from Intermediate 21) (2R,3R,4S,5R)-2-{6-[(4-Chloro-2-methylphenyl)amino]-9H-purin-9-yl}-5-ethynyltetrahydrofuran-3,4-diol

¹H NMR (DMSO) δ9.69 (1H,s,NH); 8.61(1H,s,CH); 8.45 (1H,s,CH); 7.61 (1H,d,CH); 7.58 (1 H,d,CH); 7.48d (1H,dd,CH); 6.19 (1H,d,CH); 5.98 (1.2H, v.br.s.,2xOH); 5.03 (1 H,t,CH); 4.78 (1 H,dd,CH); 4.59 (1H,t,CH); 3.96 (1H,d,CH); 2.40 (3H,s,CH₃).
LC/MS Rt = 2.85min.
Mass Spectrum *m*/*z* 386 [MH+].

### Example 40: (2R,3R,4S,5R)-2-{6-[(5-Chloro-2-methylphenyl)amino]-9H-purin-9-yl}-5-ethynyltetrahydrofuran-3,4-diol

### Intermediate 22: (2R,3R,4R,5R)-4-(acetyloxy)-2-{6-[(5-chloro-2-methylphenyl)amino]-9H-purin-9-yl}-5-ethynyltetrahydrofuran-3-yl acetate

Starting materials: (2R,3R,4R,5R)-4-(acetyloxy)-2-(6-chloro-9H-purin-9-yl)-5-ethynyltetrahydrofuran-3-yl acetate and 5-chloro-2-methylaniline.

### Example 40: (from Intermediate 22) (2R,3R,4S,5R)-2-{6-[(5-chloro-2-methylphenyl)amino]-9H-purin-9-yl}-5-ethynyltetrahydrofuran-3,4-diol

LC/MS Rt = 2.86min.
Mass Spectrum m/z 386 [MH+].

### Example 41: (2R,3R,4S,5R)-2-{6-[(2-Bromo-4-fluorophenyl)amino]-9H-purin-9-yl}-5-ethynyltetrahydrofuran-3,4-diol

### Intermediate 23: (2R,3R,4R,5R)-4-(acetyloxy)-2-{6-[(2-bromo-4-fluorophenyl)amino]-9H-purin-9-yl}-5-ethynyltetrahydrofuran-3-yl acetate

Starting materials: (2R,3R,4R,5R)-4-(acetyloxy)-2-(6-chloro-9H-purin-9-yl)-5-ethynyltetrahydrofuran-3-yl acetate and 2-bromo-4-fluoroaniline.

### Example 41: (from Intermediate 23) (2R,3R,4S,5R)-2-{6-[(2-bromo-4-fluorophenyl)amino]-9H-purin-9-yl}-5-ethynyltetrahydrofuran-3,4-diol

LC/MS Rt = 2.79min.
Mass Spectrum m/z 435 [MH+].

### Example 42: (2R,3R,4S,5R)-2-{2-chloro-6-[(3-fluoro-2-methylphenyl)amino]-9H-purin-9-yl}-5-ethynyltetrahydrofuran-3,4-diol

### Intermediate 24:

### (2R,3R,4R,5R)-4-(Acetyloxy)-2-{2-chloro-6-[(3-fluoro-2-methylphenyl)amino]-9H-purin-9-yl}-5-ethynyltetrahydrofuran-3-yl acetate

Starting materials: (2R,3R,4R,5R)-4-(acetyloxy)-2-(6-chloro-9H-purin-9-yl)-5-ethynyltetrahydrofuran-3-yl acetate and 3-fluoro-2-methylaniline.

### Example 42: (from Intermediate 24) (2R,3R,4S,5R)-2-{2-chloro-6-[(3-fluoro-2-methylphenyl)amino]-9H-purin-9-yl}-5-ethynyltetrahydrofuran-3,4-diol

LC/MS Rt = 3.17min.
Mass Spectrum m/z 405 [MH+].

### Example 43: (2R,3R,4S,5R)-2-{6-[(3,4-Difluorophenyl)amino]-9H-purin-9-yl}-5-ethynyltetrahydrofuran-3,4-diol

### Intermediate 25: (2R,3R,4R,5R)-4-(acetyloxy)-2-{6-[(3,4-difluorophenyl)amino]-9H-purin-9-yl}-5-ethynyltetrahydrofuran-3-yl acetate

Starting materials: (2R,3R,4R,5R)-4-(acetyloxy)-2-(6-chloro-9H-purin-9-yl)-5-ethynyltetrahydrofuran-3-yl acetate and 3,4-difluoroaniline.

### Example 43: (from Intermediate 25) (2R,3R,4S,5R)-2-{6-[(3,4-Difluorophenyl)amino]-9H-purin-9-yl}-5-ethynyltetrahydrofuran-3,4-diol

¹H NMR (MeOH) δ 8.5 (1 H,s,CH); 8.41 (1H,s,CH); 8.14 (1H,m,CH); 7.52 (1 H,m,CH); 7.4 (1H,m,CH); 6.2 (1 H,d,CH); 4.91 (1 H,m,CH); 4.78 (1H,m,CH); 4.49 (1H,t,CH); 3.31 (1H,d,CH).
LC/MS Rt = 2.85min.
Mass Spectrum m/z 374 [MH+].

### Example 44: (2R,3R,4S,5R)-2-{6-[(2-methylphenyl)amino]-9H-purin-9-yl}-6-ethynyltetrahydrofuran-3,4-diol

### Intermediate26: (2R,3R,4R,5R)-4-(Acetyloxy)-2-{6-[(2-methylphenyl)amino]-9H-purin-9-yl}-5-ethynyltetrahydrofuran-3-yl acetate

Starting materials: (2R,3R,4R,5R)-4-(acetyloxy)-2-(6-chloro-9H-purin-9-yl)-5-ethynyltetrahydrofuran-3-yl acetate and 2-methyl aniline

### Example 44: (from intermediate26): (2R,3R,4S,5R)-2-{6-[(2-methylphenyl)amino]-9H-purin-9-yl}-5-ethynyltetrahydrofuran-3,4-diol

LC/MS Rt = 3.05 min.
Mass Spectrum m/z 352 [MH+].

### Examale 45: (2R,3R,4S,5R)-2-{6-[(4-methylphenyl)amino]-9H-purin-9-yl}-5-ethynyltetrahydrofuran-3,4-diol

### Intermediate 27: (2R,3R,4R,5R)-4-(acetyloxy)-2-{6-[(4-methylphenyl)amino]-9H-purin-9-yl}-5-ethynyltetrahydrofuran-3-yl acetate

Starting materials: (2R,3R,4R,5R)-4-(acetyloxy)-2-(6-chloro-9H-purin-9-yl)-5-ethynyltetrahydrofuran-3-yl acetate and 4-methyl aniline

### Example 45: (from intermediate 27) (2R,3R,4S,5R)-2-{6-[(4-methylphenyl)amino]-9H-purin-9-yl}-5-ethynyltetrahydrofuran-3,4-diol

LC/MS Rt = 3.26 min.
Mass Spectrum m/z 352 [MH+].

### Example 46: (2R,3R,4S,5R)-2-{6-[(3-Chloro-2-fluorophenyl)amino]-9H-purin-9-yl}-5-ethynyltetrahydrofuran-3,4-diol

### Intermediate28: (2R,3R,4R,5R)-4-(acetyloxy)-2-{6-[(3-chloro-2-fluorophenyl)amino]-9H-purin-9-yl}-5-ethynyltetrahydrofuran-3-yl acetate

Starting materials: (2R,3R,4R,5R)-4-(acetyloxy)-2-(6-chloro-9H-purin-9-yl)-5-ethynyltetrahydrofuran-3-yl acetate and 3-chloro-2-fluoro aniline

### Example 46 (from intermediate 28) (2R,3R,4S,5R)-2-{6-[(3-chloro-2-fluorophenyl)amino]-9H-purin-9-yl}-5-ethynyltetrahydrofuran-3,4-diol

LC/MS Rt = 2.83min.
Mass Spectrum m/z 390 [MH+].

### Example 47:(2R,3R,4S,5R)-2-{6-[(2-Fluoro-5-methylphenyl)amino]-9H-purin-9-yl}-5-ethynyltetrahydrofuran-3,4-diol;

### Intermediate 29: (2R,3R,4R,5R)-4-(Acetyloxy)-2-{6-[(2-fluoro-5-methylphenyl)amino]-9H-purin-9-yl}-5-ethynyltetrahydrofuran-3-yl acetate

Starting materials: (2R,3R,4R,5R)-4-(acetyloxy)-2-(6-chloro-9H-purin-9-yl)-5-ethynyltetrahydrofuran-3-yl acetate and 2-fluoro-5-methyl aniline

### Example 47: (from intermediate 29) (2R,3R,4S,5R)-2-{6-[(2-fluoro-5-methylphenyl)amino]-9H-purin-9-yl}-5-ethynyltetrahydrofuran-3,4-diol

LC/MS Rt = 2.72min.
Mass Spectrum m/z 369 [MH+].

### LC/MS System

- Column: 3.3cm x 4.6mm ID, 3um ABZ+PLUS
- Flow Rate: 3ml/min
- Injection Volume: 5µl
- Temp: RT

- Solvents:: A: 0.1% Formic Acid + 10mMolar Ammonium Acetate. B: 95% Acetonitrile + 0.05% Formic Acid

| **Gradient** | Time | A% | B% |
|---|---|---|---|
| | 0.00 | 100 | 0 |
| | 0.70 | 100 | 0 |
| | 4.20 | 0 | 100 |
| | 5.30 | 0 | 100 |
| | 5.50 | 100 | 0 |

### Automated preparative HPLC.

- Column: 10cm x 21.2mm ID, 5um ABZ+PLUS
- Flow Rate: 4ml/min
- Temp: RT

- Solvents:: A: HPLC water + 0.1% Formic Acid B: Acetonitrile + 0.05% Formic Acid

| **Gradient** | Time | A% | B% |
|---|---|---|---|
| | 0.00 | 95 | 5 |
| | 1.45 | 95 | 5 |
| | 20 | 10 | 90 |
| | 30 | 10 | 90 |
| | 32 | 95 | 5 |

### Reporter Gene Experiments in veast

Agonist activity at human adenosine A1 receptors was measured in yeast cell lines expressing the adenosine A1 receptor together with a chimeric G-protein which linked receptor stimulation to the expression of the enzyme β-galactosidase. Cells were plated out in 96-well plates in culture medium to which was added 2mM 3-AT (3-aminotriazole) to limit basal growth in the absence of agonist stimulation, and FDG (fluorescein di-β-D-galactopyranoside) as substrate for β-galactosidase, which converts FDG to fluorescein. For measurement of potency, agonists were added to the appropriate wells at a concentration range of approximately 10⁻¹⁰ - 10⁻⁵M and incubated at 30°C for 18 hours. At this point the amount of fluorescein generated was measured in a spectrophotometer. From these readings, the concentration-dependence of the stimulation by the agonist can be calculated. One of the agonists tested on each 96-well plate was the standard non-selective agonist, N-ethylcarboxamidoadenosine (NECA), and the potency of all test agonists is expressed relative to that of the NECA standard.

(ECR = equipotent concentration ratio relative to NECA = 1)

### Reporter Gene Experiments in CHO cells

Agonist activity at human adenosine A3 receptors was measured in Chinese hamster ovary (CHO) cells containing the CRE/SPAP/HYG (CRE = cyclic AMP response element; HYG = hygromycin resistance; SPAP = secreted placental alkaline phosphatase) reporter gene elements, which upon stimulation of cAMP levels produced SPAP. A cell line was used which was stably transfected with the human adenosine A3 receptor in addition to the above elements. Cells were plated out in 96-well plates in culture medium and incubated at 37°C for 1 hour. For measurement of potency, agonists were added to the appropriate wells at a concentration range of approximately 10⁻¹⁰ - 10⁻⁵M. 15Min later, cAMP levels were stimulated by addition of a maximal concentration of forskolin. All cells were then incubated for a further 5 hours at 37°C, and cooled to room temperature, after which a substrate for the phosphatase (para-nitrophenol phosphate, pNPP), which is converted by SPAP to a coloured reagent) was then added and the 96-well plates were read in a plate reader. From these readings, the concentration-dependence of the inhibition by the agonist for forskolin-stimulated SPAP production can be calculated. As with the yeast assays, one of the agonists tested on each 96-well plate was the standard non-selective agonist, N-ethylcarboxamidoadenosine (NECA), and the potency of all test agonists is expressed relative to that of the NECA standard.

**Table 2:**

| Potencies in the reporter gene assay | | |
|---|---|---|
| **Example No.** | **Adenosine A1 receptor ECR*** | **Adenosine A3 receptor ECR*** |
| 33 | 26.31 | >215 |
| 34 | 39 | >607 |
| 1 | 9.95 | >1070 |
| 2 | 10 | >3580 |
| 3 | 6.48 | >823 |
| 35 | 6.48 | >974 |
| 36 | 7.15 | >1117 |
| 32 | 7.94 | >1070 |
| 37 | 4.79 | >145 |
| 38 | 5.45 | >145 |
| 39 | 7.36 | >168 |
| 40 | 16.94 | >505 |
| 41 | 5.92 | >505 |
| 4 | 27.96 | >407 |
| 5 | 3 | >196 |
| 6 | 24.6 | >1059 |
| 42 | 9.17 | >220 |
| 7 | 11.4 | >178 |
| 8 | 7.94 | >178 |
| 9 | 23.81 | >165 |
| 43 | 34.9 | >188 |
| 10 | 6.89 | >1066 |
| 11 | 5 | >210 |
| 12 | 4.47 | >80.2 |
| 13 | 18.86 | >386 |
| 14 | 14.86 | >502 |
| 15 | 18.79 | >778 |
| 17 | 2.88 | >416 |
| 19 | 8.57 | >3208 |
| 18 | 6.81 | >3208 |

| | | |
|---|---|---|
| *ECR = equipotent concentration ratio relative to NECA = 1 (see description in Reporter Gene Assay). | | |

Throughout the specification and the claims which follow, unless the context requires otherwise, the word 'comprise', and variations such as 'comprises' and 'comprising', will be understood to imply the inclusion of a stated integer or step or group of integers but not to the exclusion of any other integer or step or group of integers or steps.

## Claims

1. A compound of formula (Ia): 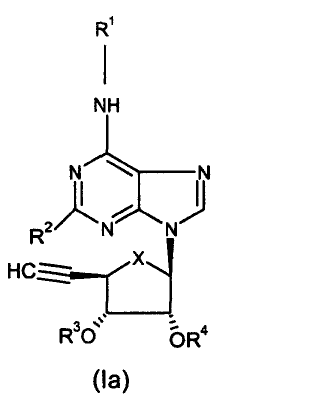 wherein X represents O or CH₂;
R¹ represents:
(i) -(alk)ₙ-(C₃₋₉)cycloalkyl or -(alk)ₙ-(C₃₋₉)cycloalkenyl, said cycloalkyl or cycloalkenyl group being optionally substituted by one or more substituents selected from OH, halogen, C₁₋₆alkyl, -C₁₋₆alkoxy, C_{2- 6}alkenyloxy-, C₂₋₆alkynyloxy- and phenyl, wherein (alk) represents C₁₋₃alkyl and n represents 0 or 1, and said (alk) group may be optionally substituted by a C₃₋₆cycloalkyl group;
(ii) a phenyl group optionally substituted by one or more substituents selected from: halogen, CF₃, cyano, -C₁₋₆alkyl, -C₂₋₆alkenyl, -C_{2- 6}alkynyl, C₁₋₆alkoxy-, -C₁₋₆alkylOH, -CO₂H and -CO₂C₁₋₆ alkyl;
(iii) a C₄₋₇heterocyclic group containing at least one heteroatom selected from O, N or S, and optionally substituted by one or more substituents selected from: OH, -C₁₋₆alkyl, -C₁₋₆alkoxy, -CO₂C_{1- 4}alkyl, -COC₁₋₄alkyl, -CO₂aryl or -CO₂(alk)ₙ(C₃₋₆)cycloalkyl, wherein (alk) represents C₁₋₃alkyl and n represents 0 or 1;
(iv) a straight or branched C₁₋₁₂alkyl group optionally substituted by one or more groups selected from phenyl, halogen, hydroxy, and C₃₋₇ cycloalkyl, wherein one or more carbon atoms of the C₁₋₁₂alkyl group may be optionally replaced by a group independently selected from S(=O)ₙ (where n is 0, 1 or 2) and N;
(v) a fused bicyclic ring 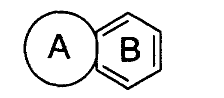
wherein A represents C₄₋₆cycloalkyl or phenyl and B represents phenyl optionally substituted by C₁₋₃alkyl, and the bicyclic ring is attached to the purine-6-amino moiety via a ring atom of ring A;
R² represents -C₁₋₃alkyl, halogen, hydrogen or -C₁₋₃alkoxy group;
R³ and R⁴ independently represent H or a -C₁₋₆alkyl group;
or a pharmaceutically acceptable salt and/or solvate thereof.

2. A compound according to claim 1 wherein R² represents hydrogen or halogen.

3. A compound according to claim 1 or claim 2 wherein R³ and R⁴ each represent hydrogen.

4. A compound according to any one of claims 1 to 3 wherein X represents O.

5. A compound according to claim 1 selected from:
(2R,3R,4S,5R)-2-{6-[(4-chloro-2-fluorophenyl)amino]-9H-purin-9-yl}-5-ethynyltetrahydrofuran-3,4-diol;
(2R,3R,4S,5R)-2-{2-chloro-6-[(4-chloro-2-fluorophenyl)amino]-9H-purin-9-yl}-5-ethynyltetrahydrofuran-3,4-diol;
(2R,3R,4S,5R)-2-{6-[(2-chloro-4-fluorophenyl)amino]-9H-purin-9-yl}-5-ethynyltetrahydrofuran-3,4-diol;
(2R,3R,4S,5R)-5-ethynyl-2-{6-[(4-fluoro-2-methylphenyl)amino]-9H-purin-9-yl}tetrahydrofuran-3,4-diol;
(2R,3R,4S,5R)-2-{6-[(2,4-difluorophenyl)amino]-9H-purin-9-yl}-5-ethynyltetrahydrofuran-3,4-diol;
(2R,3R,4S,5R)-5-ethynyl-2-{6-[(3-fluoro-2-methylphenyl)amino]-9H-purin-9-yl}tetrahydrofuran-3,4-diol;
(2R,3R,4S,5R)-2-{6-[(3-chloro-2-methylphenyl)amino]-9H-purin-9-yl}-5-ethynyltetrahydrofuran-3,4-diol;
(2R,3R,4S,5R)-2-{6-[(4-chloro-2-methylphenyl)amino]-9H-purin-9-yl}-5-ethynyltetrahydrofuran-3,4-diol;
(2R,3R,4S,5R)-2-{6-[(5-chloro-2-methylphenyl)amino]-9H-purin-9-yl}-5-ethynyltetrahydrofuran-3,4-diol;
(2R,3R,4S,5R)-2-{6-[(2-bromo-4-fluorophenyl)amino]-9H-purin-9-yl}-5-ethynyltetrahydrofuran-3,4-diol;
(2R,3R,4S,5R)-2-{2-chloro-6-[(3-fluoro-2-methylphenyl)amino]-9H-purin-9-yl}-5-ethynyltetrahydrofuran-3,4-diol;
(2R,3R,4S,5R)-2-{6-[(3,4-difluorophenyl)amino]-9H-purin-9-yl}-5-ethynyltetrahydrofuran-3,4-diol;
N-ethyl-2-({9-[(2R,3R,45,5R)-5-ethynyl-3,4-dihydroxytetrahydrofuran-2-yl]-9H-purin-6-yl}amino)ethanesulfonamide;
ethyl 4-({9-[(2R,3R,4S,5R)-5-ethynyl-3,4-dihydroxytetrahydrofuran-2-yl]-9H-purin-6-yl}amino)piperidine-1-carboxylate;
(2R,3R,4S,5R)-5-ethynyl-2-(6-{[(1S,2S)-2-hydroxycyclopentyl]amino}-9H-purin-9-yl)tetrahydrofuran-3,4-diol;
(2R,3R,4S,5R)-2-[6-indan-2-ylamino)-9H-purin-9-yl]-5-ethynyltetrahyd rofu ran-3,4-d iol;
(2R,3R,4S,5R)-2-[6-(cyclobutylamino)-9H-purin-9-yl]-5-ethynyltetrahydrofuran-3,4-diol;
(2R,3R,4S,5R)-5-ethynyl-2-{6-[(2-phenylethyl)amino]-9H-purin-9-yl}tetrahydrofuran-3,4-diol;
(2R,3R,4S,5R)-2-[6-(cyclohexylamino)-9H-purin-9-yl]-5-ethynyltetrahydrofuran-3,4-diol;
(2R,3R,4S,5R)-5-ethynyl-2-(6-{[(1S*,2S*,3S*,4R*)-3-methylbicyclo[2.2.1]hept-2-yl]amino}-9H-purin-9-yl)tetrahydrofuran-3,4-diol;
(2R,3R,4S,5R)-5-ethynyl-2-(6-{[(1R*,*2S)-2-methoxy-2-phenylcyclopentyl]amino}-9H-purin-9-yl)tetrahydrofuran-3,4-diol;
(2R,3R,4S,5R)-2-[6-(cyclopropylamino)-9H-purin-9-yl]-5-ethynyltetrahydrofuran-3,4-diol;
(2R,3R,4S,5R)-2-{6-[(cyclopropylmethyl)amino]-9H-purin-9-yl}-5-ethynyltetrahydrofuran-3,4-diol;
(2R,3R,4S,5R)-2-[6-(cyclopentylamino)-9H-purin-9-yl]-5-ethynyltetrahydrofuran-3,4-diol;
(2R,3R,4S,5R)-5-ethynyl-2-(6-{[(2R,3R)-2-methyltetrahydrofuran-3-yl]amino}-9H-purin-9-yl)tetrahydrofuran-3,4-diol;
(2R,3R,4S,5R)-5-ethynyl-2-(6-{[(2S,3S)-2-methyltetrahydrofuran-3-yl]amino}-9H-purin-9-yl)tetrahydrofuran-3,4-diol;
(2R,3R,4S,5R)-5-ethynyl-2-{6-[(trans-4-methoxycyclohexyl)amino]-9H-purin-9-yl}tetrahydrofuran-3,4-diol;
2R,3R,4S,5R)-5-ethynyl-2-{6-[(trans-4-ethoxycyclohexyl)amino]-9H-purin-9-yl}tetrahydrofuran-3,4-diol;
2R,3R,4S,5R)-5-ethynyl-2-{6-[(trans-4-propenyloxycyclohexyl)amino]-9H-purin-9-yl}tetrahydrofuran-3,4-diol;
(2R,3R,4S,5R)-5-ethynyl-2-[6-(tetrahydro-2H-pyran-4-ylamino)-9H-purin-9-yl]tetrahydrofuran-3,4-diol;
(2R,3R,4S,5R)-2-{6-[(1R*,5R*,6S*)-bicyclo[3.2.0]hept-6-ylamino]-9H-purin-9-yl}-5-ethynyltetrahydrofuran-3,4-diol;
(2R,3R,4S,5R)-2-{6-[(2,2-dimethylcyclopropyl)amino]-9H-purin-9-yl}-5-ethynyltetrahydrofuran-3,4-diol;
(2R,3R,4S,5R)-5-ethynyl-2-{6-[(trans-4-hydroxycyclohexyl)amino]-9H-purin-9-yl}tetrahydrofuran-3,4-diol;
(2R,3R,4S,5R)-2-{6-[(2-methylphenyl)amino]-9H-purin-9-yl}-5-ethynyltetrahydrofuran-3,4-diol;
(2R,3R,4S,5R)-2-{6-[(4-methylphenyl)amino]-9H-purin-9-yl}-5-ethynyltetrahydrofuran-3,4-diol;
(2R,3R,4S,5R)-2-{6-[(3-chloro-2-fluorophenyl)amino]-9H-purin-9-yl}-5-ethynyltetrahydrofuran-3,4-diol;
(2R,3R,4S,5R)-2-{6-[(2-fluoro-5-methylphenyl)amino]-9H-purin-9-yl}-5-ethynyltetrahydrofuran-3,4-diol;
butyl 4-({9-[(2R,3R,4S,5R)-5-ethynyl-3,4-dihydroxytetrahydrofuran-2-yl]-9H-purin-6-yl}amino)piperidine-1-carboxylate;
cyclopropylmethyl 4-({9-[(2R,3R,4S,5R)-5-ethynyl-3,4-dihydroxytetrahydrofuran-2-yl]-9H-purin-6-yl}amino)piperidine-1-carboxylate;
(2R,3R,4S,5R)-5-ethynyl-2-{(6-[(1S,2S)-2-methoxycyclopentyl]amino}-9H-purin-9-yl)tetrahydrofuran-3,4-diol;
(2R,3R,4S,5R)-5-ethynyl-2-{6-[(4,4-difluoro)cyclohexyl]amino}-9H-purin-9-yl)tetrahydrofuran-3,4-diol;
(2R,3R,4S,5R)-5-ethynyl-2-{6-[(cyclohex-3-enyl)amino]}-9H-purin-9-yl)tetrahydrofuran-3,4-diol;
(2R,3R,4S,5R)-5-ethynyl-2-[6-(dicyclopropylmethyl)amino]-9H-purin-9-yl)tetrahydrofuran-3,4-diol;
(2R,3R,4S,5R)-5-ethynyl-2-[6-(cyclooctyl)amino]-9H-purin-9-yl)tetrahydrofuran-3,4-diol;
(2R,3R,4S,5R)-5-ethynyl-2-[6-(cycloheptyl)amino]-9H-purin-9-yl)tetrahydrofuran-3,4-diol;
(2R,3R,4S,5R)-5-ethynyl-2-{6-[(1R*,4S*)-4-methoxy-cyclohept-2-enylamino)-9H-purin-9-yl]-tetrahydrofuran-3,4-diol; and
(2R,3R,4S,5R)-5-ethynyl-2-{6-[(1S*,4R*)-4-methoxy-cyclohept-2-enylamino)-9H-purin-9-yl]-tetrahydrofuran-3,4-diol; or
a pharmaceutically acceptable salt and/or solvate thereof

6. A pharmaceutical composition comprising a compound according to any one of claims 1 to 5 or a pharmaceutically acceptable salt and/or solvate thereof together with a pharmaceutical carrier and/or excipient.

7. Use of a compound according to any one of claims 1 to 5 or a pharmaceutically acceptable salt and/or solvate thereof for the manufacture of a medicament for the treatment of a patient suffering from a condition where there is an advantage in decreasing plasma free fatty acid concentration, or reducing heart rate.

8. Use of a compound according to any one of claims 1 to 5 or a pharmaceutically acceptable salt and/or solvate thereof for the manufacture of a medicament for the treatment of a patient suffering from or susceptible to ischaemic heart disease, peripheral vascular disease or stroke or which subject is suffering pain, a CNS disorder, sleep apnoea or emesis.

## Patentansprüche

1. Verbindung der Formel (Ia): 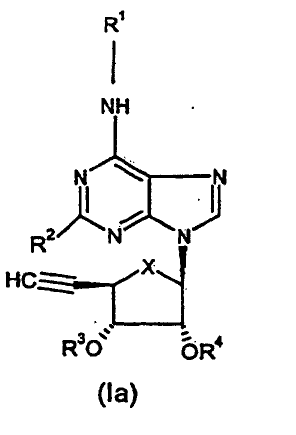 wobei X für O oder CH₂ steht;
R¹ für folgende steht:
(i) -(Alk)ₙ-(C₃₋₉)cycloalkyl oder -(Alk)ₙ-(C₃₋₉)cycloalkenyl, wobei der Cycloalkyl- oder der Cycloalkenylrest gegebenenfalls mit einem oder mehreren Substituenten, ausgewählt aus OH, Halogen, C₁₋₆-Alkyl, -C₁₋₆-Alkoxy, C₂₋₆-Alkenyloxy-, C₂₋₆-Alkinyloxy- und Phenyl, substituiert ist, wobei (Alk) für C₁₋₃-Alkyl steht und n für 0 oder 1 steht und der (Alk)-Rest gegebenenfalls mit einem C₃₋₆-Cycloalkylrest substituiert sein kann;
(ii) einen Phenylrest, gegebenenfalls substituiert mit einem oder mehreren Substituenten ausgewählt aus Halogen, CF₃, Cyano, -C₁₋₆-Alkyl, -C₂₋₆-Alkenyl, -C₂₋₆-Alkinyl, C₁₋₆-Alkoxy-, -C₁₋₆-AlkylOH, -CO₂H und -CO₂C₁₋₆-Alkyl;
(iii) einen C₄₋₇ heterocyclischen Rest, der mindestens ein Heteroatom, ausgewählt aus O, N oder S, enthält und gegebenenfalls mit einem oder mehreren Substituenten, ausgewählt aus OH, -C₁₋₆-Alkyl, -C₁₋₆-Alkoxy, -CO₂C₁₋₄-Alkyl, -COC₁₋₄-Alkyl, -CO₂-Aryl oder -CO₂(Ak)ₙ(C₃₋₆)cycloalkyl, substituiert ist, wobei (Alk) für C₁₋₃-Alkyl steht und n für 0 oder 1 steht;
(iv) einen geraden oder verzweigten C₁₋₁₂-Alkylrest, gegebenenfalls substituiert mit einem oder mehreren Resten ausgewählt aus Phenyl, Halogen, Hydroxy und C₃₋₇-Cycloalkyl, wobei ein oder mehrere Kohlenstoffatome des C₁₋₁₂-Alkylrests gegebenenfalls durch einen Rest unabhängig ausgewählt aus S(=O)ₙ (wobei n für 0, 1 oder 2 steht) und N ersetzt sein können;
(v) einen kondensierten bicyclischen Ring 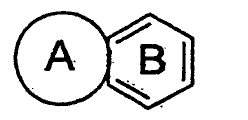
wobei A für C₄₋₆-Cycloalkyl oder Phenyl steht und B für Phenyl, gegebenenfalls substituiert mit C₁₋₃-Alkyl, steht und der bicyclische Ring über ein Ringatom von Ring A an die Purin-6-amino-Einheit gebunden ist;
R² für -C₁₋₃-Alkyl, Halogen, Wasserstoff oder einen -C₁₋₃-Alkoxyrest steht; und
R³ und R⁴ unabhängig voneinander für H oder einen -C₁₋₆Alkylrest stehen;
oder ein pharmazeutisch verträgliches Salz und/oder Solvat davon.

2. Verbindung nach Anspruch 1, wobei R² für Wasserstoff oder Halogen steht.

3. Verbindung nach Anspruch 1 oder Anspruch 2, wobei R³ und R⁴ jeweils für Wasserstoff stehen.

4. Verbindung nach einem der Ansprüche 1 bis 3, wobei X für O steht.

5. Verbindung nach Anspruch 1, ausgewählt aus:
(2R,3R,4S,5R)-2-{6-[(4-Chlor-2-fluorphenyl)amino]-9H-purin-9-yl}-5-ethinyltetrahydrofuran-3,4-diol;
(2R,3R,4S,5R)-2-{2-Chlor-6-[(4-chlor-2-fluorphenyl)amino]-9H-purin-9-yl}-5-ethinyltetrahydrofuran-3,4-diol;
(2R,3R,4S,5R)-2- {6-[(2-Chlor-4-fluorphenyl)amino]-9H-purin-9-yl}-5-ethinyltetrahydrofuran-3,4-diol;
(2R,3R,4S,5R)-5-Ethinyl-2-{6-[(4-fluor-2-methylphenyl)amino]-9H-purin-9-yl }tetrahydrofuran-3,4-diol;
(2R,3R,4S,5R)-2- {6-[(2,4-Difluorphenyl)amino]-9H-purin-9-yl}-5-ethinyltetrahydrofuran-3,4-diol;
(2R,3R,4S,5R)-5-Ethinyl-2-{6-[(3-fluor-2-methylphenyl)amino]-9H-purin-9-yl}tetrahydrofuran-3,4-diol;
(2R,3R,4S,5R)-2-{6-[(3-Chlor-2-methylphenyl)amino]-9H-purin-9-yl}-5-ethinyltetrahydrofuran-3,4-diol;
(2R,3R,4S,5R)-2-{6-[(4-Chlor-2-methylphenyl)amino]-9H-purin-9-yl}-5-ethinyltetrahydrofuran-3,4-diol;
(2R,3R,4S,5R)-2-{6-[(5-Chlor-2-methylphenyl)amino]-9H-purin-9-yl}-5-ethinyltetrahydrofuran-3,4-diol;
(2R,3R,4S,5R)-2-{6-[(2-Brom-4-fluorphenyl)amino]-9H-purin-9-yl}-5-ethinyltetrahydrofuran-3,4-diol;
(2R,3R,4S,5R)-2-{2-Chlor-6-[(3-fluor-2-methylphenyl)amino]-9H-purin-9-yl}-5-ethinyltetrahydrofuran-3,4-diol;
(2R,3R,4S,5R)-2- {6-[(3,4-Difluorphenyl)amino]-9H-purin-9-yl}-5-ethinyltetrahydrofuran-3,4-diol;
N-Ethyl-2-({9-[(2R,3R,4S,5R)-5-ethinyl-3,4-dihydroxytetrahydrofuran-2-yl]-9H-purin-6-yl}amino)ethansulfonamid;
-4-({9-[(2R,3R,4S,5R)-5-Ethinyl-3,4-dihydroxytetrahydrofuran-2-yl]-9H-purin-6-yl}amino)piperidin-1-carbonsäureethylester;
(2R,3R,4S,5R)-5-Ethinyl-2-(6-{[(1S,2S)-2-hydroxycyclopentyl]amino}-9H-purin-9-yl)tetrahydrofuran-3,4-diol;
(2R,3R,4S,5R)-2-[6-Indan-2-ylamino)-9H-purin-9-yl]-5-ethinyltetrahydrofuran-3,4-diol; (2R,3R,4S,5R)-2-[6-(Cyclobutylamino)-9H-purin-9-yl]-5-ethinyltetrahydrofuran-3,4-diol;
(2R,3R,4S,5R)-5-Ethinyl-2-{6-[(2-phenylethyl)amino]-9H-purin-9-yl}tetrahydrofuran-3,4-diol;
(2R,3R,4S,5R)-2-[6-(Cyclohexylamino)-9H-purin-9-yl]-5-ethinyltetrahydrofuran-3,4-diol;
(2R,3R,4S,5R)-5-Ethinyl-2-(6-{[(1S*,2S*,3S*,4R*)-3-methylbicyclo[2.2.1]hept-2-yl]amino}-9H-purin-9-yl)tetrahydrofuran-3,4-diol;
(2R,3R,4S,5R)-5-Ethinyl-2-(6-{[(1R*,2S*)-2-methoxy-2-phenylcyclopentyl]amino}-9H-purin-9-yl)tetrahydrofuran-3,4-diol;
(2R,3R,4S,5R)-2-[6-(Cyclopropylamino)-9H-purin-9-yl]-5-ethinyltetrahydrofuran-3,4-diol;
(2R,3R,4S,5R)-2-{6-[(Cyclopropylmethyl)amino]-9H-purin-9-yl}-5-ethinyltetrahydrofuran-3,4-diol;
(2R,3R,4S,5R)-2-[6-(Cyclopentylamino)-9H-purin-9-yl]-5-ethinyltetrahydrofuran-3,4-diol;
(2R,3R,4S,5R)-5-Ethinyl-2-(6-{[(2R,3R)-2-methyltetrahydrofuran-3-yl]amino}-9H-purin-9-yl)tetrahydrofuran-3,4-diol;
(2R,3R,4S,5R)-5-Ethinyl-2-(6-{[(2S,3S)-2-methyltetrahydrofuran-3-yl]amino}-9H-purin-9-yl)tetrahydrofuran-3,4-diol;
(2R,3R,4S,5R)-5-Ethinyl-2-{6-[(trans-4-methoxycyclohexyl)amino]-9H-purin-9-yl}tetrahydrofuran-3,4-diol;
(2R,3R,4S,5R)-5-Ethinyl-2-{6-[(trans-4-ethoxycyclohexyl)amino]-9H-purin-9-yl }tetrahydrofuran-3,4-diol;
(2R,3R,4S,5R)-5-Ethinyl-2-{6-[(trans-4-propenyloxycyclohexyl)amino]-9H-purin-9-yl}tetrahydrofuran-3,4-diol;
(2R,3R,4S,5R)-5-Ethinyl-2-[6-(tetrahydro-2H-pyran-4-ylamino)-9H-purin-9-yl]tetrahydrofuran-3,4-diol;
(2R,3R,4S,5R)-2-{6-[(1R*,5R*,6S*)-Bicyclo[3.2.0]hept-6-ylamino]-9H-purin-9-yl}-5-ethinyltetrahydrofuran-3,4-diol;
(2R,3R,4S,5R)-2-{6-[(2,2-Dimethylcyclopropyl)amino]-9H-purin-9-yl}-5-ethinyltetrahydrofuran-3,4-diol;
(2R,3R,4S,5R)-5-Ethinyl-2-{6-[(trans-4-hydroxycyclohexyl)amino]-9H-purin-9-yl}tetrahydrofuran-3,4-diol;
(2R,3R,4S,5R)-2-{6-[(2-Methylphenyl)amino]-9H-purin-9-yl}-5-ethinyltetrahydrofuran-3,4-diol;
(2R,3R,4S,5R)-2-{6-[(4-Methylphenyl)amino]-9H-purin-9-yl}-5-ethinyltetrahydrofuran-3,4-diol;
(2R,3R,4S,5R)-2-{6-[(3-Chlor-2-fluorphenyl)amino]-9H-purin-9-yl}-5-ethinyltetrahydrofuran-3,4-diol;
(2R,3R,4S,5R)-2-{6-[(2-Fluor-5-methylphenyl)amino]-9H-purin-9-yl}-5-ethinyltetrahydrofuran-3,4-diol;
-4-({9-[(2R,3R,4S,5R)-5-Ethinyl-3,4-dihydroxytetrahydrofuran-2-yl]-9H-purin-6-yl}amino)piperidin-1-carbonsäurebutylester;
-4-({9-[(2R,3R,4S,5R)-5-Ethinyl-3,4-dihydroxytetrahydrofuran-2-yl]-9H-purin-6-yl }amino)piperidin-1-carbonsäurecyclopropylmethylester;
(2R,3R,4S,5R)-5-Ethinyl-2-{(6-[(1S,2S)-2-methoxycyclopentyl]amino}-9H-purin-9-yl)tetrahydrofuran-3,4-diol;
(2R,3R,4S,5R)-5-Ethinyl-2-{6-[(4,4-difluor)cyclohexyl]amino}-9H-purin-9-yl)tetrahydrofuran-3,4-diol;
(2R,3R,4S,5R)-5-Ethinyl-2- {6-[(cyclohex-3-enyl)amino]}-9H-purin-9-yl)tetrahydrofuran-3,4-diol;
(2R,3R,4S,5R)-5-Ethinyl-2-[6-(dicyclopropylmethyl)amino]-9H-purin-9-yl)tetrahydrofuran-3,4-diol;
(2R,3R,4S,5R)-5-Ethinyl-2-[6-(cyclooctyl)amino]-9H-purin-9-yl)tetrahydrofuran-3,4-diol;
(2R,3R,4S,5R)-5-Ethinyl-2-[6-(cycloheptyl)amino]-9H-purin-9-yl)tetrahydrofuran-3,4-diol;
(2R,3R,4S,5R)-5-Ethinyl-2-{6-[(1R*,4S*)-4-methoxycyclohept-2-enylamino)-9H-purin-9-yl]tetrahydrofuran-3,4-diol; und
(2R,3R,4S,5R)-5-Ethinyl-2-{6-[(1S*,4R*)-4-methoxycyclohept-2-enylamino)-9H-purin-9-yl]-tetrahydrofuran-3,4-diol; oder
einem pharmazeutisch verträglichen Salz und/oder Solvat davon.

6. Arzneimittel umfassend eine Verbindung nach einem der Ansprüche 1 bis 5 oder ein pharmazeutisch verträgliches Salz und/oder Solvat davon zusammen mit einem pharmazeutischen Träger und/oder Hilfsstoff.

7. Verwendung einer Verbindung nach einem der Ansprüche 1 bis 5 oder ein pharmazeutisch verträgliches Salz und/oder Solvat davon zur Herstellung eines Medikaments zur Behandlung eines Patienten, der an einem Zustand leidet, bei dem es vorteilhaft ist, die Konzentration an freier Fettsäure im Plasma zu verringern oder die Herzfrequenz zu reduzieren.

8. Verwendung einer Verbindung nach einem der Ansprüche 1 bis 5 oder ein pharmazeutisch verträgliches Salz und/oder Solvat davon zur Herstellung eines Medikaments zur Behandlung eines Patienten, der an koronarer Herzkrankheit, peripherer Gefäßerkrankung oder Schlaganfall leidet oder dafür anfällig ist oder wobei der Patient unter Schmerz, einer ZNS-Störung, Schlafapnoe oder Erbrechen leidet.

## Revendications

1. Composé de formule (Ia) : 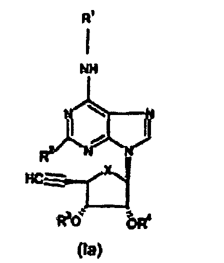 dans lequel X représente un atome de O ou un groupe CH₂ ;
R¹ représente :
(i) un groupe -(alk)ₙ-(cycloalkyle en C₃ à C₉) ou -(alk)ₙ-(cycloalcényle en C₃ à C₉), ledit groupe cycloalkyle ou cycloalcényle étant facultativement substitué avec un ou plusieurs substituants choisis entre des substituants OH, halogéno, alkyle en C₁ à C₆, -alkoxy en C₁ à C₆, alcényloxy- en C₂ à C₆, alcynyloxy- en C₂ à C₆ et phényle, (alk) représentant un groupe alkyle en C₁ à C₃ et n étant égal à 0 ou 1, et ledit groupe (alk) pouvant être facultativement substitué avec un groupe cycloalkyle en C₃ à C₆ ;
(ii) un groupe phényle facultativement substitué avec un ou plusieurs substituants à choisir entre des substituants : halogéno, CF₃, cyano, -alkyle en C₁ à C₆, -alcényle en C₂ à C₆, -alcynyle en C₂ à C₆, alkoxy- en C₁ à C₆, -alkylOH en C₁ à C₆, -CO₂H et -CO₂(alkyle en C₁ à C₆)
(iii) un groupe hétérocyclique en C₄ à C₇ contenant au moins un hétéroatome choisi entre O, N et S, et facultativement substitué avec un ou plusieurs substituants choisis entre des substituants : OH, -alkyle en C₁ à C₆, -alkoxy en C₁ à C₆, -CO₂(alkyle en C₁ à C₄), -CO(alkyle en C₁ à C₄), -CO₂aryl ou -CO₂(alk)ₙ-(cycloalkyle en C₃ à C₆), dans lequel (alk) représente un groupe alkyle en C₁ à C₃ et n est égal à 0 ou 1 ;
(iv) un groupe alkyle en C₁ à C₁₂ droit ou ramifié facultativement substitué avec un ou plusieurs groupes choisis entre des groupes phényle, halogéno, hydroxy et cycloalkyle en C₃ à C₇, un ou plusieurs atomes de carbone du groupe alkyle en C₁ à C₁₂ pouvant être facultativement remplacés par un groupe choisi indépendamment entre S(=O)ₙ (dans lequel n est égal à 0, 1 ou 2) et N ;
(v) un noyau bicyclique condensé 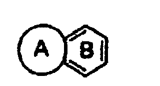
dans lequel A représente un groupe cycloalkyle en C₄ à C₆
ou phényle et B représente un groupe phényle facultativement substitué avec un substituant alkyle en C₁ à C₃, et le noyau bicyclique est fixé au groupement purine-6-amino par un atome de noyau du noyau A ;
R² représente un groupe -alkyle en C₁ à C₃, un atome d'halogène, un atome d'hydrogène ou un groupe -alkoxy en C₁ à C₃ ;
R³ et R⁴ représentent indépendamment H ou un groupe -alkyle en C₁ à C₆ ;
ou un de ses sels et/ou produits de solvatation pharmaceutiquement acceptables.

2. Composé suivant la revendication 1, dans lequel R² représente un atome d'hydrogène ou un atome d'halogène.

3. Composé suivant la revendication 1 ou la revendication 2, dans lequel R³ et R⁴ représentent chacun un atome d'hydrogène.

4. Composé suivant l'une quelconque des revendications 1 à 3, dans lequel X représente un atome de O.

5. Composé suivant la revendication 1, choisi entre :
(2R,3R,4S,5R)-2-{6-[(4-chloro-2-fluorophényl)amino]-9H-purine-9-yl}-5-éthynyltétrahydrofuranne-3,4-diol ;
(2R,3R,4S,5R)-2-{2-chloro-6-[(4-chloro-2-fluorophényl)-amino]-9H-purine-9-yl}-5-éthynyltétrahydrofuranne-3,4-diol ;
(2R,3R,4S,5R)-2-{6([(2-chloro-4-fluorophényl)amino]-9H-purine-9-yl}-5-éthynyltétrahydrofuranne-3,4-diol ;
(2R,3R,4S,5R)-5-éthynyl-2-{6-[(4-fluoro-2-méthylphényl)-amino]-9H-purine-9-yl}tétrahydrofuranne-3,4-diol ;
(2R,3R,4S,5R)-2-{6-[(2,4-difluorophényl)amino]-9H-purine-9-yl}-5-éthynyltétrahydrofuranne-3,4-diol ;
(2R,3R,4S,5R)-5-éthynyl-2-{6-[(3-fluoro-2-méthylphényl)-amino]-9H-purine-9-yl}tétrahydrofuranne-3,4-diol ;
(2R,3R,4S,5R)-2-{6-[(3-chloro-2-méthylphényl)amino]-9H-purine-9-yl}-5-éthynyltétrahydrofuranne-3,4-diol ;
(2R,3R,4S,5R)-2-{6-[(4-chloro-2-méthylphényl)amino]-9H-purine-9-yl}-5-éthynyltétrahydrofuranne-3,4-diol ;
(2R,3R,4S,5R)-2-{6-[(5-chloro-2-méthylphényl)amino]-9H-purine-9-yl}-5-éthynyltétrahydrofuranne-3,4-diol ;
(2R,3R,4S,5R)-2-{6-[(2-bromo-4-fluorophényl)amino]-9H-purine-9-yl}-5-éthynyltétrahydrofuranne-3,4-diol ;
(2R,3R,4S,5R)-2-{2-chloro-6-[(3-fluoro-2-méthylphényl)-amino]-9H-purine-9-yl}-5-éthynyltétrahydrofuranne-3,4-diol ;
(2R,3R,4S,5R)-2-{6-[(3,4-difluorophényl)amino]-9H-purine-9-yl}-5-éthynyltétrahydrofuranne-3,4-diol ;
N-éthyl-2-({9-[(2R,3R,4S,5R)-5-éthynyl-3,4-dihydroxy-tétrahydrofuranne-2-yl]-9H-purine-6-yl}amino)éthane-sulfonamide ;
4-({9-[(2R,3R,4S,5R)-5-éthynyl-3,4-dihydroxytétrahydrofuranne-2-yl]-9H(purine-6-yl}amino)pipéridine-1-carboxylate d'éthyle ;
(2R,3R,4S,5R)-5-éthynyl-2-(6-{[(1S,2S)-2-hydroxycyclopentyl]-amino}-9H-purine-9-yl)tétrahydrofuranne-3,4-diol ;
(2R,3R,4S,5R)-2-[6-indan-2-ylamino)-9H-purine-9-yl]-5-éthynyltétrahydrofuranne-3,4-diol ;
(2R,3R,4S,5R)-2-[6-(cyclobutylamino)-9H-purine-9-yl]-5-éthynyltétrahydrofuranne-3,4-diol ;
(2R,3R,4S,5R)-5-éthynyl-2-{6-[(2-phényléthyl)amino]-9H-purine-9-yl}tétrahydrofuranne-3,4-diol ;
(2R,3R,4S,5R)-2-[6-(cyclohexylamino)-9H-purine-9-yl]-5-éthynyltétrahydrofuranne-3,4-diol ;
(2R,3R,4S,5R)-5-éthynyl-2-(6-{[(1S*,2S*,3S*,4R*)-3-méthyl-bicyclo[2.2.1]hept-2-yl]amino}-9H-purine-9-yl)tétrahydrofuranne-3,4-diol ;
(2R,3R,4S,5R)-5-éthynyl-2-(6-{[(1R*,*2S)-2-méthoxy-2-phénylcyclopentyl]amino}-9H-purine-9-yl)tétrahydrofuranne-3,4-diol ;
(2R,3R,4S,5R)-2-[6-(cyclopropylamino)-9H-purine-9-yl]-5-éthynyltétrahydrofuranne-3,4-diol ;
(2R,3R,4S,5R)-2-{6-(cyclopropylméthyl)amino]-9H-purine-9-yl}-5-éthynyltétrahydrofuranne-3,4-diol ;
(2R,3R,4S,5R)-2-[6-(cyclopentylamino)-9H-purine-9-yl]-5-éthynyltétrahydrofuranne-3,4-diol ;
(2R,3R,4S,5R)-5-éthynyl-2-(6-{[(2R,3R)-2-méthyl-tétrahydrofuranne-3-yl]amino}-9H-purine-9-yl)tétrahydrofuranne-3,4-diol ;
(2R,3R,4S,5R)-5-éthynyl-2-(6-{[(2S,3S)-2-méthyl-tétra-hydrofuranne-3-yl]amino}-9H-purine-9-yl)tétrahydrofuranne-3,4-diol ;
(2R,3R,4S,5R)-5-éthynyl-2-{6-[(trans-4-méthoxycyclohexyl)-amino]-9H-purine-9-yl}tétrahydrofuranne-3,4-diol ;
(2R,3R,4S,5R)-5-éthynyl-2-{6-[(trans-4-éthoxyclohexyl)-amino]-9H-purine-9-yl}tétrahydrofuranne-3,4-diol ;
(2R,3R,4S,5R)-5-éthynyl-2-{6-[(trans-4-propényloxycyclohexyl)-amino]-9H-purine-9-yl}tétrahydrofuranne-3,4-diol ;
(2R,3R,4S,5R)-5-éthynyl-2-[6-(tétrahydro-2H-pyranne-4-ylamino)-9H-purine-9-yl]tétrahydrofuranne-3,4-diol ;
(2R,3R,4S,5R)-2-{6-[(1R*,5R*,6S*)-bicyclo[3.2.0]hept-6-ylamino]-9H-purine-9-yl}-5-éthynyltétrahydrofuranne-3,4-diol ;
(2R,3R,4S,5R)-2-{6-[(2,2-diméthylcyclopropyl)amino]-9H-purine-9-yl]-5-éthynyltétrahydrofuranne-3,4-diol ;
(2R,3R,4S,5R)-5-éthynyl-2-{6-[(trans-4-hydroxycyclohexyl)-amino]-9H-purine-9-yl}tétrahydrofuranne-3,4-diol ;
(2R,3R,4S,5R)-2-{6-[(2-méthylphényl)amino]-9H-purine-9-yl}-5-éthynyltétrahydrofuranne-3,4-diol ;
(2R,3R,4S,5R)-2-{6-[(4-méthylphényl)amino]-9H-purine-9-yl}-5-éthynyltétrahydrofuranne-3,4-diol ;
(2R,3R,4S,5R)-2-{6-[(3-chloro-2-fluorophényl)amino]-9H-purine-9-yl}-5-éthynyltétrahydrofuranne-3,4-diol ;
(2R,3R,4S,5R)-2-{6-[(2-fluoro-5-méthylphényl)amino]-9H-purine-9-yl}-5-éthynyltétrahydrofuranne-3,4-diol ;
4-({9-[(2R,3R,4S,5R)-5-éthynyl-3,4-dihydroxytétrahydrofuranne-2-yl]-9H-purine-6-yl}amino)pipéridine-1-carboxylate de butyle ;
4-({9-[(2R,3R,4S,5R)-5-éthynyl-3,4-dihydroxytétrahydrofuranne-2-yl]-9H-purine-6-yl}amino)pipéridine-1-carboxylate de cyclopropylméthyle ;
(2R,3R,4S,5R)-5-éthynyl-2-{(6-[(1S,2S)-2-méthoxycyclopentyl]-amino}-9H-purine-9-yl)tétrahydrofuranne-3,4-diol ;
(2R,3R,4S,5R)-5-éthynyl-2-{6-[(4,4-difluoro)cyclohexyl]-amino}-9H-purine-9-yl)tétrahydrofuranne-3,4-diol ;
(2R,3R,4S,5R)-5-éthynyl-2-{6-[(cyclohex-3-enyl)amino]}-9H-purine-9-yl)tétrahydrofuranne-3,4-diol ;
(2R,3R,4S,5R)-5-éthynyl-2-[6-(dicyclopropylméthyl)amino]-9H-purine-9-yl)tétrahydrofuranne-3,4-diol ;
(2R,3R,4S,5R)-5-éthynyl-2-[6-(cyclooctyl)amino]-9H-purine-9-yl)tétrahydrofuranne-3,4-diol ;
(2R,3R,4S,5R)-5-éthynyl-2-[6-(cycloheptyl)amino]-9H-purine-9-yl)tétrahydrofuranne-3,4-diol ;
(2R,3R,4S,5R)-5-éthynyl-2-{6-[(1R*,4S*)-4-méthoxy-cyclohept-2-énylamino)-9H-purine-9-yl]-tétrahydrofuranne-3,4-diol ; et
(2R,3R,4S,5R)-5-éthynyl-2-{6-[(1S*,4R*)-4-méthoxy-cyclohept-2-énylamino)-9H-purine-9-yl]tétrahydrofuranne-3,4-diol ; ou
un de ses sels et/ou produits de solvatation pharmaceutiquement acceptable.

6. Composition pharmaceutique comprenant un composé suivant l'une quelconque des revendications 1 à 5 ou un de ses sels et/ou produits de solvatation pharmaceutiquement acceptables, conjointement avec un support et/ou excipient pharmaceutique.

7. Utilisation d'un composé suivant l'une quelconque des revendications 1 à 5 ou d'un de ses sels et/ou produits de solvatation pharmaceutiquement acceptables pour la production d'un médicament destiné au traitement d'un patient souffrant d'une affection pour laquelle il existe un avantage à réduire la concentration plasmatique d'acides gras libres, ou de réduire la fréquence cardiaque.

8. Utilisation d'un composé suivant l'une quelconque des revendications 1 à 5 ou d'un de ses sels et/ou produits de solvatation pharmaceutiquement acceptables pour la production d'un médicament destiné au traitement d'un patient souffrant ou susceptible de souffrir d'une maladie cardiaque ischémique, d'une maladie vasculaire périphérique ou d'un ictus, ou bien d'un sujet souffrant d'une douleur, d'un trouble du SNC, de l'apnée du sommeil ou de vomissements.
